# EUROPEAN PATENT APPLICATION

(11) **EP 4 506 452 A1**
(43) Date of publication of application: **12.02.2025**
(21) Application number: 23190240.4
(22) Date of filing: 08.08.2023
(51) Int. Cl.: C12N 9/12, C12N 15/63, C12N 15/85, C12N 15/90

(54) **TRANSPOSASE WITH IMPROVED BIOSAFETY AND EFFICIENCY**

(71) Applicant: Max-Delbrück-Centrum für Molekulare Medizin in der Helmholtz-Gemeinschaft, 13125 Berlin (DE)
(72) Inventor: Raskó, Tamás, 16321 Bernau (DE); Pille, Jan, 13355 Berlin (DE); Izsvák, Zsuzsanna, 13125 Berlin (DE)
(74) Representative: Doll, Markus Alexander

(57) **Abstract**

The invention relates to the field of transposases. In particular, it provides a polypeptide comprising or consisting of a Tc1/*mariner* superfamily transposase, e.g., a Sleeping Beauty transposase, which comprises a nuclear export signal and a chaperone-mediated autophagy signal. The transposases of the invention were found to exhibit reduced half-life and thus enhanced biosafety. The invention further provides a sequence-optimized RNA comprising a 5'UTR and a 3'UTR that encodes a transposase with high transposition efficiency. The transposase encoded by the RNA may be, e.g., a transposase of the invention that is characterized by a reduced half-life while retaining full integration efficiency. The invention also provides vectors and cells for the expression of said transposase and/or RNA, kits and pharmaceutical compositions comprising said transposase and/or RNA, e.g., for use in gene therapy, e.g., adoptive T cell therapy, or uses and methods for gene delivery into the genome of a cell.

## Description

The invention relates to the field of transposases. In particular, it provides a polypeptide comprising or consisting of a *Tc1*/*mariner* superfamily transposase, e.g., a Sleeping Beauty transposase, which comprises a nuclear export signal and a chaperone-mediated autophagy signal. The transposases of the invention were found to exhibit reduced half-life and thus enhanced biosafety. The invention further provides a sequence-optimized RNA comprising a 5'UTR and a 3'UTR that encodes a transposase with high transposition efficiency. The transposase encoded by the RNA may be, e.g., a transposase of the invention that is characterized by a reduced half-life while retaining full integration efficiency. The invention also provides vectors and cells for the expression of said transposase and/or RNA, kits and pharmaceutical compositions comprising said transposase and/or RNA, e.g., for use in gene therapy, e.g., adoptive T cell therapy, or uses and methods for gene delivery into the genome of a cell.

Gene therapies are associated with great and growing expectations, as they have the inherent potential to cure a wide range of diseases. The success of a gene therapy depends not only on its simplicity, cost-efficiency and scalability, but also to a decisive extent on the safety and efficiency of the gene transfer system used.

The non-viral Sleeping Beauty (SB) transposon system is comparatively well characterized and consists of a transposon and a transposase. Originally, i.e., in the natural system, the gene coding for the transposase is located between so-called "inverted terminal repeats" (ITRs) on the transposon. Once expressed, the transposase is able to bind to these ITRs to catalyze the excision of the transposon from the donor DNA locus and its reintegration adjacent to a TA dinucleotide base pair in a recipient DNA sequence. By exchanging the transposase gene with a cassette comprising a gene of interest (GOI) and the provision of the transposase in trans either as DNA, mRNA, or protein, the SB technology became an efficient and universally applicable gene transfer system. The physical separation of transposon and transposase provides room for a wide variety of optimizations and opens up a multitude of possible applications in various cell types of vertebrates (including humans). Indeed, the SB transposon system is also increasingly being used for the development of gene therapies, of which first approaches are already in clinical development. This has brought the aspect of biological safety more and more into focus, also on the part of the regulatory authorities.

To avoid undesirable genotoxic effects, a gene transfer system should not remain in the cell nucleus for longer than necessary, i.e., in case a transposase is being used, it should be removed as quickly as possible after successful transposition. Otherwise, in the presence of an active transposase, an already integrated GOI could be removed or remobilized, which is associated with an increased risk of genomic instability. Moreover, the SB transposase is sensitive to aggregation and its accumulation due to, e.g., its overexpression, induces toxicity.

Accordingly, there is a clear need for optimizing the half-life of transposases, an issue which, however, has not yet been approached by the measures described in the prior art so far.

In light of this, the inventors addressed the problem of providing advantageous variants of transposases commonly used in gene therapy that are characterized by a reduced half-life and thus an improved biosafety.

The problem is solved by the present invention, in particular by the subject matter of the claims.

In particular, the present invention provides a polypeptide capable of mobilizing a transposon that comprises a nuclear export signal and a chaperone-mediated autophagy signal.

Transposons (also referred herein as transposable elements or simply TEs) are DNA sequences that have the ability to move their genetic information within the genome. TEs can be classified into two groups. Class I TE are so-called retrotransposons that follow a copy-and-paste mechanism and use an RNA intermediate for this process (Wicker et al. 2007). Class II transposons rely solely on DNA intermediates for their transposition process. In this category subclass I transposons follow a cut-and-paste mechanism, during which the transposon is excised from one genomic location and reintegrates at a different location (Wicker et al. 2007).

In a preferred embodiment, the polypeptide according to the invention is capable of mobilizing a transposon of the *Tc1*/*mariner* superfamily, i.e., the polypeptide comprises a Tc1/mariner superfamily transposase.

The *Tc1*/*mariner* superfamily of transposons follows the canonical cut-and-paste mechanism of Class II subclass I transposons. The TEs of this superfamily are flanked by inverted terminal repeats (ITRs) and harbor a gene encoding a transposase, which is an enzymatic factor that catalyzes the transposition reaction (Wicker et al. 2007). The transposase binds to the ITRs, excises the TE from the donor locus and reintegrates it adjacent to a TA target sequence, which ultimately leads to a TA target site duplication (Wicker et al. 2007). Accordingly, the term "transposase" as used herein refers to an enzyme that is, as a component of a functional nucleic acid-protein complex, capable of mediating a transposition reaction. The term "transposition reaction" as used herein refers to a reaction where a transposon inserts into a target nucleic acid.

Preferably, the transposase is active in human cells. For instance, it may be a Sleeping Beauty, Frog Prince, Minos, ZB, Tdr1 or Passport transposase. In a preferred embodiment, the polypeptide of the invention comprises a Sleeping Beauty transposase.

The Sleeping Beauty (SB) transposon was reconstructed from fossil DNA sequences within fish genomes and was the first DNA transposon shown to be active in vertebrates (Ivics et al., 1997). It is widely used as a genetic engineering tool in various preclinical studies and clinical trials (Amberger et al., 2020). Since the discovery of the SB transposon, several mutations were identified leading to an overall higher integration efficiency. These mutations culminated in the currently most active transposase variant of SB called SB100X (Mátés et al., 2009). The structural and biochemical features of the SB transposase catalysing the transposition reaction are of special interest, because based on the enzymatic activity of the transposase, the efficiency of SB transposon integration into target cell genomes can be enhanced. The SB transposase comprises an N-terminal DNA binding domain (amino acids [aa] 1-110) and a C-terminal catalytic domain (DDE; aa 114-340) (Ivics et al., 1997). Both domains are connected by a flexible linker region that harbors a nuclear localization signal (NLS; aa 97-123) (Ivics et al., 1997). The DNA binding domain consists of two subdomains, the PAI and RED subdomain, each of them forming a helix-turn-helix (HTH) motif important for the recognition and binding of transposon DNA (Ivics et al., 1997, Izsvák et al., 2002). The catalytic domain, with its three conserved amino acids in the catalytic center (D153, D244, E279 (DDE)), catalyzes DNA hydrolysis reactions, which are required for excision and transesterification taking place in the integration reaction (lzsvák et al., 2002, Montaño et al., 2011, Yang et al., 2006).

Other transposases of the Tc1/mariner superfamily, e.g., Frog Prince, have a similar structure.

Preferably, the polypeptide according to the invention thus comprises a Sleeping Beauty transposase having at least 80% amino acid identity to SEQ ID NO: 43 (SB100X), optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 43.

In another embodiment, the polypeptide according to the invention does not comprise a Sleeping Beauty transposase but a different member of the Tc1/mariner superfamily of transposases. It may, for instance, comprise an FP (Frog Prince) transposase having at least 80% amino acid identity to SEQ ID NO: 44, optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 44.

Alternatively, the polypeptide according to the invention can comprise a Minos transposase having at least 80% amino acid identity to SEQ ID NO: 45, optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 45.

The polypeptide according to the invention can also comprise a ZB transposase having at least 80% amino acid identity to SEQ ID NO: 46, optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 46.

Alternatively, the polypeptide according to the invention can comprise a Tdr1 transposase having at least 80% amino acid identity to SEQ ID NO: 47, optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 47.

In yet another embodiment, the polypeptide according to the invention can comprise a Passport transposase having at least 80% amino acid identity to SEQ ID NO: 48, optionally, at least 90% sequence identity, at least 95% sequence identity, at least 98% sequence identity or at least 99% sequence identity. Optionally, there are only one, two or three amino acid differences to said sequence. The polypeptide according to the invention may also comprise an amino acid sequence having 100% sequence identity to SEQ ID NO: 48.

The respective transposases are classified by their amino acid identity to the respective "wildtype" transposase sequence. That is, if the transposase has an amino acid identity of at least 80% to any of the recited sequences, and sequence identity to one of said sequences is highest, it is classified as a transposon of said class.

The polypeptide according to the invention preferably comprises a hyperactive variant of a transposase, i.e., a transposase comprising mutations that result in a transpositional activity which is enhanced (i.e., by more than 100%, preferably more than 110%, more than 120%, more than 150% or about 200% or more) compared to a "wild type" version of any of the transposases described herein. In the context of the invention, the terms "transposition efficiency" and "transpositional activity" are used synonymously and refer to the activity of a given transposase that can be assessed in a transposition reaction. Suitable experimental set-ups are described in the experimental section herein or may use the classical binary transposition assay as described in Ivics et al., 1997.

Many mutations at various positions have been identified that are capable of enhancing the transpositional activity of transposases, most of which were demonstrated to even further elevate the transpositional activity of hyperactive SB100X. For instance, WO2009/003671, which is herewith incorporated by reference, discloses hyperactive variants of transposases that comprise substitutions or groups of substitutions that enhance one or more property, in particular the transpositional activity of the transposase. Thus, in one embodiment, the polypeptide according to the invention comprises a hyperactive variant of any of the herein described transposases, preferably a hyperactive variant of an SB transposase, comprising at least one of the following substitutions or groups of substitutions:
(1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
(2) K14R//R214D/K215A/E216V/N217Q;
(3) K33A/R115H/R214D/K215A/E216V/N217Q/M243H;
(4) K14R/K30R/A205K/H207V/K208R/D210E/R214D/K215A/E216V/N217Q/M243H;
(5) K13D/K33A/T83N/H207V/K208R/D210E/M243Q;
(6) K13A/K33A/R214D/K215A/E216V/N217Q;
(7) K33A/T83N/R214D/K215NE216V/N217Q//G317E;
(8) K14R/T83A/M243Q;
(9) K14R/T83A/I100L/M243Q;
(10) K14R/T83A/R143L/M243Q;
(11) K14R/T83A/R147E/M243Q;
(12) K14R/T83A/M243Q/E267D;
(13) K14R/T83A/M243Q/T314N;
(14) K14R/K30R/I110L/A205K/H207V/K208R/D210E/R214D/K215A/ E216V/N217Q/M243H;
(15) K14R/K30R/R143L/A205K/H207V/K208R/D210E/R214D/K215A/ E216V/N217Q//M243H;
(16) K14R/K30R/R147E/A205K/H207V/K208R/D210E/R214D/K215A/ E216V/N217Q/M243H;
(17) K14R/K30R/A205K/H207V/K208R/D210E/R214D/K215A/ E216V/N217Q//M243H/E267D;
(18) K14R/K30R/A205K/H207V/K208R/D210E/R214D/K215A/ E216V/N217Q/M243H/T314N;
(19) K14R/K30R/A205K/H207V/K208R/D210E/R214D/K215A/ E216V/N217Q//M243H/G317E;
(20) K14R/K33A/R115H/R214D/K215A/E216V/N217Q/M243H;
(21) K14R/K30R/R147E/A205K/H207V/K208R/D210E/R214D/K215A/ E216V/N217Q//M243H/T314N;
(22) K14R/K30R/R143U/A205K/H207V/K208R/D210E/R214D/K215A/ E216V/N217Q//M243H/E267D;
(23) K14R/K30R/R143L/A205K/H207V/K208R/D210E/R214D/K215A/ E216V/N217Q//M243H/T314N;
(24) K14R/K30R/R143L/A205K/H207V/K208R/D210E/R214D/K215A/ E216V/N217Q//M243H/G317E;
(25) K14R/K33A/R115H/R143L/R214D/K215A/E216V/N217Q/M243H;
(26) K14R/K33A/R115H/R147E//R214D/K215A/E216V/N217Q/M243H;
(27) K14R/K33A/R115H/R214D/K215A/E216V/N217Q//M243H/E267D;
(28) K14R/K33A/R115H/R214D/K215A/E216V/N217Q//M243H/M314N;
(29) K14R/K33A/R115H/R214D/K215A/E216V/N217Q//M243H/G317E;
(30) K14R/T83A/M243Q/G317E; or
(31) K13A/K33A/T83N/R214D/K215A/E216V/N217Q;

All amino acid "positions" used in the context of the invention refer to the amino acid sequence of SB transposase of SEQ ID NO: 43 and are determined in relation to the transposase portion of the polypeptide only. The skilled person will easily be able to determine the corresponding positions of the outlined substitution(s) or groups of substitutions in the amino acid sequences of the other naturally occurring transposases described herein, e.g., by alignment.

The polypeptide according to the invention may further comprise any of the substitutions disclosed in European patent application 22 200 543.1, i.e., it may comprise a substitution in position 124. In case the transposase is SB or Tdr1 transposase, the original or "wildtype" amino acid residue in position 124 is Q. For ZB, it is N, for FP it is S, for Minos it is S and for Passport it is K. The amino acid substituting the wild type amino acid residue in position 124 may be selected from the group consisting of C, A, R, N, D, E, G, H, I, L, K, M, F, P, S, T, W, Y and V. In one embodiment, the substitute amino acid in position 124 is C, A, R, D, E, G, H, I, L, M, F, P, S, T, W, Y and V. The substitute amino acid in position 124 may also be, e.g., C, A, D, E, G, H, I, L, M, F, P, W, Y and V.

If the transposase, as preferred in the context of the invention, is a SB transposase such as SB100X, the substitute amino acid in position 124 can be C, A, R, N, D, E, G, H, I, L, K, M, F, P, S, T, W, Y and V. Preferred amino acids in said context are C, S or M, most preferably, C. The same applies for a Tdr1 transposase.

If the transposase is a ZB transposase, the substitute amino acid in position 124 can be C, A, R, D, E, G, H, I, L, K, M, F, P, S, T, W, Y and V. Preferred amino acids in said context are C, S or M, most preferably, C.

If the transposase is a FP transposase, the substitute amino acid in position 124 can be C, A, R, N, D, E, G, H, I, L, K, M, F, P, T, W, Y and V. Preferred amino acids in said context are C or M, most preferably, C.

If the transposase is a Passport transposase, the substitute amino acid in position 124 can be C, A, R, N, D, E, G, H, I, L, M, F, P, S, T, W, Y and V. Preferred amino acids in said context are C or M, most preferably, C.

As shown in European patent application 22 200 543.1, a significant increase in transpositional activity was achieved if the amino acid in position 124 is C. Therefore, in a particularly advantageous embodiment, the polypeptide of the invention comprises a transposase having a substitution leading to C in position 124.

Optionally, the polypeptide may comprise any of the transposases and variants thereof described herein and further comprises a modification as disclosed in international patent application PCT/EP2022/075007, which is herewith incorporated by reference.

As described in PCT/EP2022/075007, transposases of the Tc1/mariner superfamily comprising a substitution in any of positions 187, 247 or 248, e.g., a K248R substitution, have an improved integration pattern compared to transposases not having a substitution in said positions. They have a better safety profile, as they decrease the integration of transposons into exons as well as transcriptional regulatory regions of genes in the human genome. The transposons thus integrate into genetic safe harbours with an increased frequency. Accordingly, the polypeptide of the invention may have a gain in specificity of integration into the genome and thus comprise one or more of the following substitutions:
a) the amino acid in said substituted position 187 may be A, N, C, Q, G, I, L, M, S, V, W, K, R, E, P, T and S, preferably, V;
b) the amino acid in said substituted position 247 may be R, C, A or S; preferably, R, and/or
c) the amino acid in said substituted position 248 may be R, S, V, I or C, preferably, R.

For a substitution at position 187 in a SB transposase, a H187A, H187N, H187C, H187Q, H187G, H187I, H187L, H187M, H187S, H187V, H187W, H187K, H187R, H187E, H187P, H187T and H187S substitution is preferred, most preferably, a H187V substitution.

For a substitution at position 247 in a SB transposase, a P247R, P247C, P247A and P247S substitution is preferred, most preferably, a P247R substitution.

For a substitution at position 248 in a SB transposase, a K248R, K248S, K248V, K248I or K248C substitution is preferred, most preferably, a K248R substitution.

The polypeptide of the invention may also comprise a transposase having an improved solubility in water compared to SB of SEQ ID NO: 43, e.g., a high solubility transposase as described by Querques et al., 2009 or in EP3673053A. To improve solubility, the transposase may, e.g., further comprise at least one substitution in the amino acid position 176 and/or 212, wherein the amino acid in position 176 is not C and the amino acid in position 212 is not I, preferably, wherein the amino acid in position 176 and/or212 is S, wherein, optionally, both the amino acids in position 176 and 212 are S.

The relatively long half-life of transposases such as SB100X and its variants constitutes a biosafety problem, as a prolonged transposase activity may lead to, e.g., remobilizations of the integrated transposon and thus cause genomic instability. The present inventors therefore tested different strategies to reduce the half-life of transposases. Transposases such as SB100X are DNA modifier enzymes and therefore localize and act in the cell nucleus. One of the primary goals was therefore to (a) decrease the expressed transposase protein level in the reaction space (nucleus) and (b) facilitate the degradation of the enzymatically-active transposase levels in the whole targeted cells.

As a first measure for achieving this goal, the polypeptide according to the invention comprises a nuclear export signal. Nuclear export signals (NES) are short peptide sequences of about 8-15 amino acid residues capable of targeting an export cargo protein from the nucleus of a eukaryotic cell through the nuclear pore complex into the cytoplasm. Many different NES have been identified in the past; however, conventional NES usually exhibit a consensus amino acid sequence of Φ1-X₂₋₃-Φ2-X₂₋₃-Φ3-X-Φ4, where Φn represents a hydrophobic amino acid selected from Leu, Val, Ile, Phe, or Met and X can be any amino acid (Kutay and Güttinger, 2005, Xu et al., 2012) The hydrophobic amino acid residues of the NES mediate translocation of the protein into the cytoplasm by establishing an interaction with exportins such as, e.g., CRM1. These exportins in turn directly interact with the constituent building blocks of the nuclear pore complex, so-called nucleoporins, which are embedded in the nuclear envelope and thereby escort the cargo protein into the cytoplasm.

The NES of the polypeptide according to the invention may have an amino acid sequence that corresponds to the consensus sequence of Φ1-X₂₋₃-Φ2-X₂₋₃-Φ3-X-Φ4. Alternatively, the sequence of the NES may also deviate from said consensus sequence, e.g., it may comprise any of the expanded consensus sequences described in Kosugi et al., 2008, such as, e.g., Φ1-X₃-Φ2-X₂-Φ3-X-Φ4; Φ1-X₂-Φ2-X₂-Φ3-X-Φ4; Φ1-X₃-Φ2-X₃-Φ3-X-Φ4; Φ1-X₂-Φ2-X₃-Φ3-X-Φ4; Φ1-X-Φ2-X₂-Φ3-X-Φ4; Φ1-X₂-Φ2-X₃-Φ3-X₂-Φ4; where Φ*n* = L, V, I, F, or M, and A, C, T, and W can be one of the Φ*n*). The NES may also comprise a sequence comprising five instead of four key hydrophobic positions, because CRM1 offers five hydrophobic pockets to bind the Φ positions of the NES. Such NES sequences are disclosed, for example, in Güttler et al., 2010 or Güttlerand Görlich, 2011 and comprise an additional hydrophobic residue Φ0 at the N-terminus. Accordingly, the NES may have an amino acid sequence of Φ0-X_{0,3}-Φ1-X₃-Φ2-X_{2,3}-Φ3-X-Φ4, where Φ0 can be I, V, M, L, A, Y, F, W, or P; Φ1 can be L, I, V, M, F, A, or W; Φ2 can be F, M, L, I, V, Y, or W; Φ3 can be L, M, I, V, F, W, or A; and Φ4 can be L, I, M, V, or F. Alternatively, the NES sequence may be Φ0-Φ1ₚᵣₒ-X₁-Φ2-X₂-Φ3-X-Φ4, where Pro is strongly preferred in the Φ1 position. The NES may also have any of the consensus sequences as first described in Xu et al., 2012, such as, e.g., Φ1-X_{1,2,3}-Φ2-[^W]₂-Φ3-[^W]-Φ4, Φ1-X_{2,3}-Φ2-[^W]₃-Φ3-[^W]-Φ4 or Φ1-X₂-Φ2-X[^W]₂-Φ3-[^W]₂-Φ4, where [^W] is any of the 20 amino acids except Trp and Ala and Thr residues can be used only once at either position Φ1 or Φ2.

One skilled in the art is readily capable of selecting a suitable NES sequence to achieve successful nuclear export of the polypeptide of the invention. However, in a preferred embodiment, the NES may comprise an amino acid sequence having at least 70%, preferably at least 80%, more preferably at least 90% sequence identity to SEQ ID NO: 30 (LALKLAGLDI). Most preferably, the NES comprises an amino acid sequence having 100% sequence identity to SEQ ID NO: 30 or consists thereof.

The NES preferably forms the N-terminus of the polypeptide according to the invention, i.e., it is linked to the N-terminus of the transposase. It may, however, in alternative embodiments, also be located at other suitable positions within the polypeptide.

The polypeptide according to the invention further comprises a protein degradation signal (also known as degron), i.e., an amino acid sequence that can be recognized by a proteolytic apparatus to mediate degradation of the polypeptide. Proteins carrying a degradation signal are degraded either by the autophagy-lysosomal pathway or by ubiquitin-dependent targeting to the proteasome.

The inventors surprisingly found that the half-life of a transposase polypeptide could be reduced most effectively when the polypeptide was targeted for degradation via the autophagy lysosomal pathway rather than via the ubiquitin-proteasome system (see Examples). Accordingly, in a preferred embodiment, the protein degradation signal of the polypeptide according to the invention is a signal that targets the polypeptide according to the invention for autophagy-lysosomal degradation, i.e., it is a chaperone-mediated autophagy (CMA-) signal.

Autophagy refers to a process in which intracellular macromolecules such as proteins are transported to specialized single-membrane organelles known as lysosomes. The lysosomal lumen has an acidic pH and comprises a multitude of different hydrolases, i.e., enzymes such as proteases, lipases, glycosidases, and nucleases capable of catalysing the degradation of the macromolecular substrates. The autophagy-lysosomal pathway is normally a non-selective protein and organelle degradation process, but it may become selective, e.g., upon starvation in a process referred to as chaperone-mediated autophagy (CMA). During CMA only a subset of cytosolic soluble proteins is degraded. The selectivity of CMA is based on the presence of a specific motif in the amino acid sequence of the substrate proteins, which is recognized in the cytosol by chaperones and co-chaperones such as hsc70 (heat shock cognate protein of 70-kDa). These chaperone proteins target the CMA substrate to the lysosome and assist in substrate unfolding. Once the substrate protein has been transported to the lysosomal membrane it binds to the cytosolic tail of lysosome-associated membrane protein 2A (LAMP-2A), a glycoprotein which subsequently multimerizes into a higher order complex required for substrate translocation into the lysosomal lumen.

The specific protein motifs or -signals recognized by CMA usually consist of five amino acids and must meet the following characteristics: One amino acid of the sequence must be basic, another must be acidic, a third must be hydrophobic, and a fourth one can be either basic or hydrophobic but never acidic, whereby the exact order of these amino acids appears to be of little to no relevance (Orenstein and Cuervo, 2010). Therefore, the CMA-signal of the polypeptide according to the present invention also preferably adheres to the above-mentioned characteristics. The best-studied chaperone-mediated autophagy signal is KFERQ, which was found in the first ever identified CMA substrate, ribonuclease A. The present inventors found that use of KFERQ as a CMA-signal in combination with the addition of a NES was suitable to significantly lower the half-life of SB100X transposase. Accordingly, in a preferred embodiment, the CMA-signal of the polypeptide according to the invention has the amino acid sequence KFERQ (SEQ ID NO: 31). It may, however, also have the amino acid sequence of any of e.g., KFSRK (SEQ ID NO: 32), VKKDQ (SEQ ID NO: 33), KFFEQ (SEQ ID NO: 34), KIREI (SEQ ID NO: 35), QVELR (SEQ ID NO: 36), QYFKS (SEQ ID NO: 37), LKSFQ (SEQ ID NO: 38), LKYFQ (SEQ ID NO: 39), EKFLQ (SEQ ID NO: 40), QREFK (SEQ ID NO: 41) orVDKFQ (SEQ ID NO: 42). In particular preferred embodiments, the CMA has an amino acid sequence selected from the group comprising KFERQ (SEQ ID NO: 31), KFFEQ (SEQ ID NO: 34), QVELR (SEQ ID NO: 36), LKSFQ (SEQ ID NO: 38), LKYFQ (SEQ ID NO: 39) and EKFLQ (SEQ ID NO: 40).

It is also disclosed that the polypeptide according to the invention comprises a protein degradation signal that is not a CMA-signal, e.g., it may comprise a degradation signal that targets the polypeptide to the ubiquitin ligase machinery or to the proteasome.

The protein degradation signal, preferably, the CMA signal, may be located at or near to the N-terminus of the polypeptide, at or near to the C-terminus of the polypeptide or within the polypeptide. In a preferred embodiment, the NES and the protein degradation signal, e.g., the CMA-signal, are both located at the N-terminus of the polypeptide, i.e., they form a tag that is linked to the N-terminus of the transposase, wherein, preferably, the NES and the protein degradation signal are arranged in a manner so that the NES forms the N-terminus of the final polypeptide of the invention. In case the NES and/or the protein degradation signal, e.g., the CMA-signal, are linked to the N-terminus of the transposase, the amino acid sequence of the transposase may lack 1 to 10 N-terminal amino acids of the full length naturally occurring transposase. Preferably, the amino acid sequence of the transposase lacks a methionine at position M1. The amino acid sequence of the transposase may also lack 1 to 10 C-terminal amino acids of the full length naturally occurring transposase in case the NES and/or the protein degradation signal, e.g., the CMA-signal are linked to the C-terminus of the transposase.

The NES and the protein degradation signal, e.g., the CMA signal, may be linked to the N-terminus of the transposase via a suitable linker sequence. The linker sequence should be chosen such that it does not interfere with the proper folding of the final polypeptide into a functional transposase. It may, e.g., be 1 to 50 amino acids long. For instance, the linker sequence may comprise an amino acid sequence of SEQ ID NO: 49 (KLGGGAPAVGGGPKAADK). However, a person skilled in the art will be able to identify alternative suitable linker sequences that differ from that of SEQ ID NO: 49. In alternative embodiments, the NES and the protein degradation signal, e.g., the CMA signal, may be directly linked to the N-terminus of the polypeptide, i.e., without the need of a linker sequence.

In a preferred embodiment, the polypeptide according to the invention comprises a SB100X transposase as well as an NES and a CMA signal linked to its N-terminus via a linker sequence and may comprise an amino acid sequence having 80% sequence identity to SEQ ID NO: 50. It may optionally comprise an amino acid sequence having more than 80% sequence identity to SEQ ID NO: 50, e.g., at least 85%, at least 90%, at least, 91, at least 92, at least 93, at least 95, at least 96, at least 97, at least 98 or at least 99% sequence identity to SEQ ID NO: 50. It may also comprise an amino acid sequence having 100% sequence identity to SEQ ID NO: 50 or consist thereof.

As shown in the Examples below, the present inventors found that the addition of a NES and a CMA signal sequence significantly shortened the half-life of SB100X transposase by increasing the rate of degradation of the transposase by at least five-fold. Accordingly, the inventors successfully generated an SB100X transposase (herein also referred to as "NES-CMA-SB100X") characterized by a particularly high biosafety, as the herein described modifications minimize the risks of transposon re-integrations and unwanted accumulation of transposase protein.

However, the reduction in half-life of the transposase was also associated with a reduction in transposition efficiency.

As shown in the examples below, the NES-CMA-SB100X transposase exhibited approximately 25% lower transposition activity in mammalian cell lines and an approximately 50% lower activity in primary human immune cells compared to conventional SB100X transposase.

To take this into account, a higher amount of said transposase can be used. However, maintenance of the transposition activity is preferred.

Thus, in a quest to restore the transposition efficiency of NES-CMA-SB100X to that of SB100X, the inventors decided to optimize the expression profile of the transposase. Messenger RNA (mRNA), typically *in vitro*-transcribed, can be used as a source for transposases such as SB transposase to eliminate the risk of inadvertent integration of the transposase gene into the genome.

Accordingly, the present invention also provides an RNA encoding a polypeptide capable of mobilizing a transposon as described herein, such as, e.g., a polypeptide according to the invention, which comprise a series of modifications with positive effects on the transposition efficiency of the encoded transposase.

As a first measure, the RNA according to the invention encodes a polypeptide capable of mobilizing a transposon, wherein the RNA comprises a 5'UTR and a 3'UTR.

The eukaryotic 5'UTR, also known as leader sequence, comprises multiple cis-acting regulatory regions and binding sites for different translation initiation factors such as elF4E and eIF4G. The 5'UTR thus plays an important role in the regulation of mRNA translation.

Likewise, the 3'UTR comprises regulatory sequences that influence polyadenylation, translation efficiency, localization, and stability of the mRNA. In particular, microRNAs preferentially bind to the 3'UTR to post-transcriptionally regulate gene expression.

The present inventors tested various 5'UTRs and 3'UTRs from different sources for their ability to improve the transposition/integration efficiency of transposons encoded by the RNA of the invention. It was found that the 5'UTR of β-globin exerted a particularly advantageous effect on transfection stability. Similar effects were further observed using a synthetic 5'UTR comprising an eIF4G aptamer, i.e., a short region of about 25 to 70 nt that is bound by the translation elongation factor elF4G with elevated affinity. Exemplary aptamers are disclosed, e.g., in Miyakawa et al., 2006, the content of which is herewith incorporated by reference.

In contrast, the inventors found that a 5'UTR comprising the J-K stem-loop region of the encephalomyocarditis virus IRES as described in Terenin et al., 2013 did not produce the desired effects to the same extend as the β-globin 5'UTRs or the 5'UTR comprising an eIF4G aptamer.

Accordingly, in a preferred embodiment, the 5'UTR of the RNA according to the invention is a β-globin 5'UTR or a 5'UTR comprising an eIF4G aptamer.

For instance, the 5'UTR of the RNA according to the invention may be a β-globin 5'UTR comprising a nucleic acid sequence having at least 80%, optionally at least 85%, at least 90%, at least 95%, or at least 99% sequence identity to SEQ ID NO: 51. It may also comprise a nucleic acid sequence having 100% sequence identity to SEQ ID NO: 51 or consist thereof.

Alternatively, the 5'UTR of the RNA according to the invention may be a 5'UTR comprising an eIF4G aptamer and thus may comprise a nucleic acid sequence having at least 80%, optionally at least 85%, at least 90%, at least 95%, or at least 99% sequence identity to SEQ ID NO: 52. It may also comprise a nucleic acid sequence having 100% sequence identity to SEQ ID NO: 52 or consist thereof.

Alternatively, the 5'UTR of the RNA according to the invention may also be a synthetic 5'UTR comprising a sequence with a low predicted propensity of forming secondary structural elements such as hairpins, comprising a nucleic acid sequence having at least 80%, optionally at least 85%, at least 90%, at least 95%, or at least 99% sequence identity to SEQ ID NO: 61. It may also comprise a nucleic acid sequence having 100% sequence identity to SEQ ID NO: 61 or consist thereof.

The 3'UTR of the RNA according to the invention is preferably selected from the group consisting of one or more β-globin 3'UTRs, an α-globin 2 3'UTR, a 3'UTR comprising a Woodchuck Hepatitis Virus (WHV) Posttranscriptional Regulatory Element (WPRE), a βα-globin 3'UTR or a truncated β-globin 3'UTR.

Accordingly, in one embodiment, the RNA according to the invention comprises one or more, e.g., two or three, preferably two β-globin 3'UTRs comprising a nucleic acid sequence having at least 80%, optionally at least 85%, at least 90%, at least 91%, at least 92%, at least 93%, at least 94%, at least 95%, at least 96%, at least 97%, at least 98%, or at least 99% sequence identity to SEQ ID NO: 53. It may also comprise a 3'UTR comprising one or more nucleic acid sequences having 100% sequence identity to SEQ ID NO: 53 or consisting thereof.

In a second embodiment, the RNA according to the invention comprises α-globin 2 3'UTR comprising a nucleic acid sequence having at least 80%, optionally at least 85%, at least 90%, at least 91%, at least 92%, at least 93%, at least 94%, at least 95%, at least 96%, at least 97%, at least 98%, or at least 99% sequence identity to SEQ ID NO: 54. It may also comprise a 3'UTR comprising a nucleic acid sequence having 100% sequence identity to SEQ ID NO: 54 or consisting thereof.

In a third embodiment, the RNA according to the invention comprises a 3'UTR comprising a WPRE comprising a nucleic acid sequence having at least 80%, optionally at least 85%, at least 90%, at least 91%, at least 92%, at least 93%, at least 94%, at least 95%, at least 96%, at least 97%, at least 98%, or at least 99% sequence identity to SEQ ID NO: 55. It may also comprise a 3'UTR comprising a nucleic acid sequence having 100% sequence identity to SEQ ID NO: 55 or consisting thereof.

In a fourth embodiment, the RNA according to the invention comprises a βa-globin 3'UTR. The β- and the α-globin 3'UTRs are expected to regulate mRNA stabilty via distinct mechanisms, having potentially synergistic effects (Russell and Liebhaber, 1996). The 3'UTR may thus comprise a nucleic acid sequence having at least 80%, optionally at least 85%, at least 90%, at least 91%, at least 92%, at least 93%, at least 94%, at least 95%, at least 96%, at least 97%, at least 98%, or at least 99% sequence identity to SEQ ID NO: 56. It may also comprise a 3'UTR comprising a nucleic acid sequence having 100% sequence identity to SEQ ID NO: 56 or consisting thereof.

In a fifth embodiment, the RNA according to the invention comprises a truncated β-globin 3'UTR, i.e., a β-globin 3'UTR lacking the signal sequences for natural polyadenylation. Accordingly, the 3'UTR of the RNA according to the invention may comprise a nucleic acid sequence having at least 80%, optionally at least 85%, at least 90%, at least 91%, at least 92%, at least 93%, at least 94%, at least 95%, at least 96%, at least 97%, at least 98%, or at least 99% sequence identity to SEQ ID NO: 57. It may also comprise a 3'UTR comprising a nucleic acid sequence having 100% sequence identity to SEQ ID NO: 57 or consisting thereof.

The present inventors performed various experiments to identify RNAs comprising specific combinations of 5'UTRs and 3'UTRs disclosed herein that resulted in increased transposition efficiency of the encoded transposases, e.g., that were capable of expressing a NES-CMA-SB100X polypeptide according to the present invention that exhibited a transposition efficiency comparable to that of conventional SB100X (SEQ ID NO. 43). The most effective RNAs identified in these experiments comprised combinations of:
(a) a β-globin 5'UTR and two β-globin 3'UTRs;
(b) a β-globin 5'UTR and a truncated β-globin;
(c) a β-globin 5'UTR and an α -globin 2 3'UTR;
(d) a β-globin 5'UTR and a βa-globin 3'UTR;
(e) a 5' UTR comprising an eIF4G aptamer and two β-globin 3'UTRs.

Accordingly, the RNA to the invention preferably comprises a combination of a 5'UTR and a 3'UTR according to any of (a) to (e).

The RNA of the invention is preferably further codon-optimized for expression in the target organism and/or the target tissue. For this, isolated triplet codons of the nucleotide sequence encoding the transposase are replaced by synonymous codons in the course of in vitro mutagenesis in order to adapt the expression rate of the transposase to the preferred codon usage of the target organism or the target tissue. Codon optimization leads to an improved expression of the encoded polypeptide in a selected host organism, e.g., in a human cell. Tables for appropriately adjusting a nucleic acid sequence to the host cell's specific transcription/translation machinery are known to a skilled person. In general, it is preferred to adapt the G/C-content of the nucleotide sequence to the specific host cell conditions. For expression in human cells, an increase of the G/C content by at least 10%, more preferred at least 20%, 30%, 50%, 70% and even more preferred 90% of the maximum G/C content (coding for the respective inventive peptide variant) is preferred. Preparation and purification of such nucleic acids and/or derivatives are usually carried out by standard procedures.

The RNA according to the invention further comprises a poly(A) tail having a length of 50 to 150 nt. Poly(A) tails consist of multiple adenosine monophosphates that are added to an mRNA after transcription to protect the mRNA molecule from enzymatic degradation in the cytoplasm and to aid in transcription termination, export of the mRNA from the nucleus, and translation. Preferably, the poly(A)-tail of the RNA disclosed herein has a length of 75 to 100 nt, such as, e.g., 80 to 95 nt or 85 to 90 nt. Most preferably, the poly(A)-tail has a length of 90 nt.

The RNA according to the invention preferably is a nucleoside-modified RNA, i.e., it is a synthetic RNA comprising one or more naturally modified nucleosides or synthetic nucleoside analogues. For instance, the RNA according to the invention may comprise one or more modified nucleosides selected from the group comprising 5-methyl-CTP, N4-acetyl-CTP, Pseudo-UTP, N1-methylpseudo-UTP, 6-methoxy-UTP, 2-thio-UTP, N6-methyl-ATP or N1-methyl-ATP.

In the context of the present invention, the RNA according to the invention is considered to be nucleoside-modified if it comprises at least one modified nucleoside at any position in its nucleic acid sequence. However, in embodiments where the RNA according to the invention is a nucleoside-modified RNA, preferably at least 50% of its uridine nucleosides, cytidine nucleosides, guanosine nucleosides and/or adenosine nucleosides are substituted by a suitable corresponding modified nucleoside. More preferably, at least 60%, at least 70%, at least 75%, at least 80%, at least 85%, at least 90%, at least 95%, or at least 99% of the uridine nucleosides, cytidine nucleosides, guanosine nucleosides and/or adenosine nucleosides present in said RNA are substituted by corresponding modified nucleosides as described herein. Most preferably, the nucleoside-modified RNA is fully substituted, i.e., 100% of the uridine nucleosides, cytidine nucleosides, guanosine nucleosides and/or adenosine nucleosides present in the RNA are substituted by a suitable corresponding modified nucleoside. For instance, if the RNA according to the invention is a nucleoside-modified RNA comprising Pseudo-UTP, preferably at least 50%, at least 60%, at least 70%, at least 75%, at least 80%, at least 85%, at least 90%, at least 95%, at least 99% or, most preferably, 100% of the uridine nucleosides of the RNA are substituted by Pseudo-UTP. In the latter case, the RNA is considered to be fully substituted.

The present inventors found that, in particular, use of pseudouridine-5'-triphosphate (Pseudo-UTP), 5-Methoxyuridine-5'-triphosphate (5-Methoxy-UTP) and 5-Methylcytidine-5'-triphosphate (5-Methyl-CTP) in an RNA according to the invention allowed for the generation of a NES-CMA-SB100X transposase exhibiting stable transposon integration efficiency in primary T cells comparable to that of SB100X. Accordingly, in a preferred embodiment, the RNA of the invention comprises any one of the modified nucleosides selected from Pseudo-UTP, 5-Methoxy-UTP or 5-Methyl-CTP. The RNA may also comprise combinations of different types of modified nucleosides. For instance, it may comprise combinations of Pseudo-UTP and 5-Methoxy-UTP, Pseudo-UTP and 5-Methyl-CTP, 5-Methoxy-UTP and 5-Methyl-CTP or a combination of Pseudo-UTP, 5-Methoxy-UTP and 5-Methyl-CTP.

Preferably, the RNA according to the invention is *in* vitro-transcribed. In alternative embodiments, the RNA may however also be transcribed from a suitable vector in a cell, as described below.

The inventors used an RNA comprising the herein described modifications to express NES-CMA-SB100X polypeptides having a transposition efficiency comparable to or even higher than that of conventional SB100X transposase. Accordingly, the RNA preferably encodes a polypeptide according to the present invention, i.e., a polypeptide comprising any of the transposases described herein linked to an NES and a protein degradation signal, e.g., a CMA signal. The modifications in the RNA encoding the polypeptide of the invention allow to compensate for the loss of transposition efficiency due to the reduced half-life of the transposase.

For instance, in a first embodiment, the RNA according to the invention comprises a nucleic acid sequence of SEQ ID NO: 62 and is designated A1K. It thus encodes an NES-CMA-SB100X transposase as disclosed herein and comprises a β-globin 5'UTR and 3'UTR comprising a Woodchuck Hepatitis Virus (WHV) Posttranscriptional Regulatory Element (WPRE). The RNA may further comprise a poly(A)-tail comprising 90 adenine nucleotides and thus also consist of a nucleic acid sequence of SEQ ID NO: 4.

In another embodiment, the RNA according to the invention comprises a nucleic acid sequence of SEQ ID NO: 63 and is designated A1H. It thus encodes an NES-CMA-SB100X transposase as disclosed herein and comprises a β-globin 5'UTR and a single β-globin 3'UTR. The RNA may further comprise a poly(A)-tail comprising 90 adenine nucleotides and thus also consist of a nucleic acid sequence of SEQ ID NO: 6.

In another embodiment, the RNA according to the invention comprises a nucleic acid sequence of SEQ ID NO: 64 and is designated A1I. It thus encodes an NES-CMA-SB100X transposase as disclosed herein and comprises a β-globin 5'UTR and an α-globin 2 3'UTR. The RNA may further comprise a poly(A)-tail comprising 90 adenine nucleotides and thus also consist of a nucleic acid sequence of SEQ ID NO: 7.

In another embodiment, the RNA according to the invention comprises a nucleic acid sequence of SEQ ID NO: 65 and is designated A1O. It thus encodes an NES-CMA-SB100X transposase as disclosed herein and comprises a β-globin 5'UTR and a βa-globin 3'UTR. The RNA may further comprise a poly(A)-tail comprising 90 adenine nucleotides and thus also consist of a nucleic acid sequence of SEQ ID NO: 8.

In another embodiment, the RNA according to the invention comprises a nucleic acid sequence of SEQ ID NO: 66 and is designated A1P. It thus encodes an NES-CMA-SB100X transposase as disclosed herein and comprises a β-globin 5'UTR and a truncated β-globin 3'UTR. The RNA may further comprise a poly(A)-tail comprising 90 adenine nucleotides and thus also consist of a nucleic acid sequence of SEQ ID NO: 9.

In another embodiment, the RNA according to the invention comprises a nucleic acid sequence of SEQ ID NO: 67 and is designated B1M or of SEQ ID NO: 68 and is designated B3M. It thus encodes an NES-CMA-SB100X transposase as disclosed herein and comprises a 5'UTR comprising an eIF4G aptamer and two β-globin 3'UTRs. The RNA may further comprise a poly(A)-tail comprising 90 adenine nucleotides and thus also consist of a nucleic acid sequence of SEQ ID NO: 10 (B1M) or 11 (B3M), respectively.

In a preferred embodiment the RNA according to the invention comprises a nucleic acid sequence of SEQ ID NO: 69 and is designated A2M. It thus encodes an NES-CMA-SB100X transposase as disclosed herein and comprises a β-globin 5'UTR and two β-globin 3'UTRs. The RNA may further comprise a poly(A)-tail comprising 90 adenine nucleotides and thus may also consist of a nucleic acid sequence of SEQ ID NO: 5.

The RNA according to the invention may also comprise a nucleic acid sequence corresponding to SEQ ID NO: 69 or consist of a nucleic acid sequence corresponding to SEQ ID NO: 5, wherein each cytidine nucleoside (i.e., 100% of the cytidine nucleosides) of said RNA is substituted with 5-Methyl-CTP. The thus substituted RNA is referred to as A2M_C1 in the context of the invention.

Alternatively, the RNA may also comprise a nucleic acid sequence corresponding to SEQ ID NO: 69 or consist of a nucleic acid sequence corresponding to SEQ ID NO: 5, wherein each uridine nucleoside (i.e., 100% of the uridine nucleosides) of said RNA is subsituted by 5-Methoxy-UTP. The thus substituted RNA is designated A2M_U3 in the context of the invention.

Alternatively, the RNA may also comprise a nucleic acid sequence corresponding to SEQ ID NO: 69 or consist of a nucleic acid corresponding to SEQ ID NO: 5, wherein each uridine nucleoside (i.e., 100% of the uridine nucleosides) in said RNA is substituted by Pseudo-UTP. The thus substituted RNA is designated A2M_U1 in the context of the invention.

The RNA according to the invention does not necessarily have to encode a NES-CMA-SB100X polypeptide according to the invention. Rather, the disclosed modifications in the RNA have the potential to increase the transposition efficiency of any of the herein described transposases and their variants. Accordingly, the RNA of the invention may encode a polypeptide having at least 80% amino acid identity to any of SEQ ID NOs: 43 to 48, optionally, at least 90% sequence identity, at least 95% sequence identity, at least 98% sequence identity or at least 99% sequence identity. For instance, the RNA of the invention may encode a polypeptide having at least 80% amino acid identity to SEQ ID NO: 43 to 48 and comprising one or more of the advantageous amino acid substitutions described above. The RNA may also encode a polypeptide comprising an amino acid sequence having 100% sequence identity to SEQ ID NO: 43 to 48 or consisting thereof.

In one embodiment, the RNA according to the invention comprises, e.g., a nucleic acid sequence of SEQ ID NO: 3 and is designated SB100X A8H. It thus encodes an SB100X transposase and comprises a β-globin 5' UTR and a single β-globin 3'UTR and a poly(A)-tail comprising 90 adenine nucleotides. The RNA according to the invention may also consist of a nucleic acid sequence of SEQ ID NO: 3

Another object of the invention is a vector for introducing the RNA according to the invention or the polypeptide according to the invention into a target cell, preferably a mammalian cell. The vector may e.g., encode the RNA or polypeptide of the invention or comprise the RNA or polypeptide of the invention. In some embodiments, the vector may be, e.g., a vector that, when being expressed, results in the generation of the RNA according to the invention or the polypeptide according to the invention. It thus may be an expression vector encoding the RNA or polypeptide of the invention. Examples of such expression vectors include but are not limited to minicircles, plasmids, cosmids, phages, viruses or artificial chromosomes. In particular, a vector may be used to transport the RNA of the invention into a suitable host cell. Once in the host cell, the expression vector may replicate independently of or coincidental with the host chromosomal DNA, and several copies of the vector and its inserted DNA can be generated. In case that replication incompetent expression vectors are used - which is often the case for safety reasons - the vector may not replicate but merely direct expression of the RNA. Depending on the type of expression vector, the expression vector may be lost from the cell, i.e., only transiently expresses the RNA into protein. It may also be stable in the cell. Expression vectors typically contain expression cassettes, i.e., the necessary elements that permit transcription of the nucleic acid into an mRNA molecule.

Preferably, the vector is suitable for expression of the RNA or polypeptide of the invention in a mammalian, e.g., human cell such as a stem cell or a lymphocyte, e.g., a T lymphocyte. If the vector consists of DNA, it preferably comprises at least a regulatory region of a gene. The regulatory region may be a transcriptional regulatory region, e.g., selected from the group consisting of a promoter, an enhancer, a silencer, a locus control region, and a border element. Promoters or other expression control regions can be operably linked with the nucleic acid encoding the inventive polypeptide, e.g., to regulate expression of the polypeptide/protein in a quantitative or in a tissue-specific manner. The promotor can be a constitutive or inducible promotor.

The vector may also be suitable for introducing an already transcribed RNA, e.g., an in vitro transcribed mRNA as described herein, or a translated polypeptide according to the invention into a host cell such as a mammalian cell. Accordingly, the vector according to the invention does not have to be an expression vector encoding the RNA or polypeptide of the invention. Rather the vector can be a vehicle, such as, e.g., a nanoparticle, comprising an RNA or polypeptide of the invention which is able to direct and release said RNA or polypeptide into the target cell. For example, in a preferred embodiment, the vector may be a lipid nanoparticle comprising an RNA according to the invention. A skilled person will be aware of additional suitable vectors capable of transporting the RNA or polypeptide into a target cell.

Another object of the invention is a cell comprising the RNA of the invention and/or the polypeptide of the invention. Typically, the cell will comprise both. The cell may be a bacterial cell, e.g., in the context of plasmid propagation, but preferably it is a eukaryotic cell, in particular a mammalian cell. It may be, e.g., a human cell, a mouse cell, a rabbit cell or a rat cell. Human cells are preferred throughout the invention. The cell may be a stem cell, e.g., a pluripotent stem cell (e.g., an induced pluripotent stem cell), a hematopoietic stem cell or a lymphocyte, e.g., a T lymphocyte. For example, the cell preferably is a human cell selected from the group comprising a pluripotent stem cell or a human lymphocyte, preferably, a human T lymphocyte. The cell can also be a tumor cell.

Further, the invention provides a kit comprising
a) the polypeptide of the invention, the RNA of the invention, the vector of the invention and/or the cell of the invention; and
b) a nucleic acid comprising a cargo nucleic acid flanked by inverted terminal repeats (ITRs) of a transposon capable of being mobilized by said transposase.

Such a kit may be used for delivering the cargo nucleic acid, e.g., the RNA of the invention encoding a polypeptide according to the invention into the genome of a cell, e.g., a human cell. The cargo nucleic acid typically is an exogenous nucleic acid, i.e., a nucleic acid exogenous to the cell to which it is to be delivered. A therapeutic nucleic acid may e.g., encode a protein reduced or defective in a metabolic disease, e.g., in the context of gene therapy of said disease, a tumor suppressor gene, an immunomodulator, e.g., a cytokine, an antigen, an antibody, a T cell receptor (TCR) or chimeric antigen receptor (CAR). In a preferred embodiment, the therapeutic nucleic acid is useful for therapy of a cancer. The kit may be used for in vivo, ex vivo or in vitro applications, e.g., for use in medicine such as in cancer treatment. It may thus be a pharmaceutical kit. The cargo nucleic acid may alternatively encode a fluorescent protein and/or another selectable marker. It may also encode a protein intended to be produced in a cell, e.g., in cell culture, e.g., a hormone such as insulin.

The invention also provides a pharmaceutical composition comprising the polypeptide of the invention, the RNA of the invention, the vector of the invention, the cell of the invention, and/or the components of the kit of the invention. The pharmaceutical composition typically further comprises a pharmaceutically acceptable carrier and/or excipient. The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents, excipients, or encapsulating substances, which are suitable for administration to a subject. Possible carrier substances (e.g., diluents) are, for example, sterile water, Ringer's solution, lactated Ringer's solution, physiological saline, bacteriostatic saline (e.g., saline containing 0.9% benzyl alcohol), phosphate-buffered saline (PBS), Hank's solution, fixed oils, polyalkylene glycols, hydrogenated naphthalenes, and biocompatible lactide polymers, lactide/glycolide copolymers, or polyoxyethylene/polyoxy-propylene copolymers. In one embodiment, the carrier is PBS. The resulting solutions or suspensions are preferably isotonic to the blood of the recipient. The carrier can also be a bulking agent such as trehalose, e.g., in the context of lyophilized compositions. Suitable carriers and their formulations are described in greater detail in Remington's Pharmaceutical Sciences, 17th ed., 1985, Mack Publishing Co.

The pharmaceutical composition preferably is for use in adoptive T cell therapy or gene therapy. Gene therapy may be therapy of a metabolic or immunological disease. In one embodiment the gene therapy includes but is not limited to autologous or heterologous T cell therapy, gene therapy targeting any cell type in the blood, hematopoietic stem cell therapy, liver gene therapy, gene therapy of the central nervous system, eye gene therapy, muscle gene therapy, skin gene therapy and/or a gene therapy for treatment of cancer.

The pharmaceutical composition of the invention may also be of interest for vaccination therapy for the integration of antigens into antigen presenting cells, e.g., human professional antigen presenting cells such as dendritic cells, macrophages or B cells or their precursors. For example, specific tumor antigens, e.g. MAGE-1, may be encoded in the cargo nucleic acid for tumor vaccination or pathological antigens for the treatment of infectious diseases derived from pathogens, e.g., leprosy, tetanus, Whooping Cough, Typhoid Fever, Paratyphoid Fever, Cholera, Plague, Tuberculosis, Meningitis, Bacterial Pneumonia, Anthrax, Botulism, Bacterial Dysentry, Diarrhoea, Food Poisoning, Syphilis, Gasteroenteritis, Trench Fever, Influenza, Scarlet Fever, Diphtheria, Gonorrhoea, Toxic Shock Syndrome, Lyme Disease, Typhus Fever, Listeriosis, Peptic Ulcers, and Legionnaires' Disease; for the treatment of viral infections resulting in e.g., Acquired Immunodeficiency Syndrome, Adenoviridae Infections, Alphavirus Infections, Arbovirus Infections, Borne Disease, Bunyaviridae infections, Caliciviridae Infections, Chickenpox, Condyloma Acuminata, Coronaviridae Infections, Coxsackievirus Infections, Cytomegalovirus Infections, Dengue, DNA Virus Infections, Ecthyma, Contagious, Encephalitis, Arbovirus, Epstein-Barr Virus Infections, Erythema Infectiosum, Hantavirus Infections, Hemorrhagic Fevers, Viral, Hepatitis, Viral, Human, Herpes Simplex, Herpes Zoster, Herpes Zoster Oticus, Herpesviridae Infections, Infectious Mononucleosis, Influenza in Birds, Influenza, Human, Lassa Fever, Measles, Molluscum Contagiosum, Mumps, Paramyxoviridae Infections, Phlebotomus Fever, Polyomavirus Infections, Rabies, Respiratory Syncytial Virus Infections, Rift Valley Fever, RNA Virus Infections, Rubella, Slow Virus Diseases, Smallpox, Subacute Sclerosing Panencephalitis, Tumor Virus Infections, Warts, West Nile Fever, Virus Diseases, Yellow Fever; for the treatment of protozoological infections resulting in e.g., malaria. The subject methods may be used to deliver a wide variety of therapeutic nucleic acids.

According to the invention, a pharmaceutical composition contains an effective amount of the active agents, e.g., the polypeptide, RNA, vector, or cell described herein, to generate the desired reaction or the desired effect. A pharmaceutical composition in accordance with the present invention is preferably sterile. Pharmaceutical compositions can be provided in a uniform dosage form and may be prepared in a manner known per se. A pharmaceutical composition in accordance with the present invention may, e.g., be in the form of a solution or suspension.

Pharmaceutically acceptable carriers, adjuvants or vehicles that may be used in the compositions of this invention include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulphate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. The pharmaceutical compositions of the present invention may be administered orally, parenterally, by inhalation spray, topically, rectally, nasally, buccally, vaginally or via an implanted reservoir. The term parenteral as used herein includes subcutaneous, intravenous, intramuscular, intraarticular, intrasynovial, intrasternal, intrathecal, intrahepatic, intralesional and intracranial injection or infusion techniques. Preferably, the pharmaceutical compositions are administered orally, intraperitoneally, or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium.

The inventive pharmaceutical composition is preferably suitable for the treatment of a disease, in particular a disease caused by a gene defect such as cystic fibrosis, hypercholesterolemia, hemophilia, e.g. A, B, C or XIII, immune deficiencies including HIV, Huntington disease, α-anti-Trypsin deficiency, as well as cancer selected from colon cancer, melanomas, kidney cancer, lymphoma, acute myeloid leukemia (AML), acute lymphoid leukemia (ALL), chronic myeloid leukemia (CML), chronic lymphocytic leukemia (CLL), gastrointestinal tumors, lung cancer, gliomas, thyroid cancer, mamma carcinomas, prostate tumors, hepatomas, diverse virus-induced tumors such as e.g. papilloma virus induced carcinomas (e.g. cervix carcinoma), adeno carcinomas, herpes virus induced tumors (e.g. Burkitt's lymphoma, EBV induced B cell lymphoma), Hepatitis B induced tumors (Hepato cell carcinomas), HTLV-1 und HTLV-2 induced lymphoma, akustikus neurinoma, lung cancer, pharyngeal cancer, anal carcinoma, glioblastoma, lymphoma, rectum carcinoma, astrocytoma, brain tumors, stomach cancer, retinoblastoma, basalioma, brain metastases, medullo blastoma, vaginal cancer, pancreatic cancer, testis cancer, melanoma, bladder cancer, Hodgkin syndrome, meningeoma, Schneeberger's disease, bronchial carcinoma, pituitary cancer, mycosis fungoides, gullet cancer, breast cancer, neurinoma, spinalioma, Burkitt's lymphoma, lyryngeal cancer, thymoma, corpus carcinoma, bone cancer, non-Hodgkin lymphoma, urethra cancer, CUP-syndrome, oligodendroglioma, vulva cancer, intestinal cancer, oesphagus carcinoma, small intestine tumors, craniopharyngeoma, ovarial carcinoma, ovarian cancer, liver cancer, leukemia, or cancers of the skin or the eye; etc..

The pharmaceutical composition preferably is for treatment of a human subject. The invention also provides a method of treating a subject, e.g., a human subject in need thereof, e.g., for any of the diseases mentioned herein, comprising administering an effective amount of the pharmaceutical composition of the invention or the components of the pharmaceutical kit of the invention to said subject.

Also described is the use, e.g., an in vitro use, of the polypeptide of the invention, the RNA of the invention, the vector of the invention, the cell of the invention or the kit of the invention for introducing an exogenous nucleic acid into the genome of a cell.

Further, the invention provides a method, optionally, an in vitro method, of preparing a cell with an exogenous nucleic acid integrated into the genome of the cell, comprising steps of
a) providing to the cell a polypeptide of the invention; and
b) providing to the cell a nucleic acid comprising the exogenous nucleic acid flanked by terminal inverted repeats (TIR) of a transposon capable of being mobilized by said polypeptide.

Optionally, the polypeptide is provided to the cell by introducing the RNA of the invention into the cell. Alternatively, the polypeptide can be provided in polypeptide form, e.g., as a transposase with reduced half-life as described herein. In one embodiment the exogenous nucleic acid is comprised in the vector of the invention. It can also be administered separately.

In one embodiment, the RNA and/or the polypeptide of the invention and/or the exogenous nucleic acid is provided in the cell using a method selected from the group consisting of: electroporation, microinjection, lipoprotein particles, virus-like particles. Electroporation has been found particularly suitable for transfecting primary cells, e.g., T cells.

The invention also provides a method of preparing a protein, comprising carrying out the steps of the method for preparing a cell with an exogenous nucleic acid integrated into the genome of the cell, wherein the protein is encoded by said exogenous nucleic acid and operably linked to expression control elements such as a promotor suitable for expression in the cell.

Another object of the invention is a method of preparing the polypeptide of the invention, comprising cultivating a cell of the invention comprising the RNA of the invention and isolating the polypeptide.

Throughout the invention, the term "about" is intended to be understood as "+/- 10%". If "about" relates to a range, it refers to both lower and upper limit of the range. "A" is intended to mean "one or more", if not explicitly mentioned otherwise. All literature cited herein is herewith fully incorporated. The present invention is further illustrated, but not limited, by the following examples.

**Tab. 1: List of Sequences**

| | |
|---|---|
| SEQ ID NO: 1 | SB100X (NA) |
| SEQ ID NO: 2 | NES-CMA-SB100X (NA) |
| SEQ ID NO: 3 | SB100X A8H (NA) |
| SEQ ID NO: 4 | A1K (NA) |
| SEQ ID NO: 5 | A2M (NA) |
| SEQ ID NO: 6 | A1H (NA) |
| SEQ ID NO: 7 | A1I (NA) |
| SEQ ID NO: 8 | A1O (NA) |
| SEQ ID NO: 9 | A1P (NA) |
| SEQ ID NO: 10 | B1M (NA) |
| SEQ ID NO: 11 | B3M (NA) |
| SEQ ID NO: 12 | C2K (NA) |
| SEQ ID NO: 13 | D1K (NA) |
| SEQ ID NO: 14 | D3K (NA) |
| SEQ ID NO: 22 | A15M (NA) |
| SEQ ID NO: 23 | A16M (NA) |
| SEQ ID NO: 24 | A17M (NA) |
| SEQ ID NO: 25 | A18M (NA) |
| SEQ ID NO: 26 | A19M (NA) |
| SEQ ID NO: 27 | A20M (NA) |
| SEQ ID NO: 28 | A21M (NA) |
| SEQ ID NO: 29 | A22M (NA) |
| SEQ ID NO: 30 | Nuclear Export Signal (LALKLAGLDI) |
| SEQ ID NO: 31 | CMA-signal (KFERQ) |
| SEQ ID NO: 32 | CMA-signal (KFSRK) |
| SEQ ID NO: 33 | CMA-signal (VKKDQ) |
| SEQ ID NO: 34 | CMA-signal (KFFEQ) |
| SEQ ID NO: 35 | CMA-signal (KIREI) |
| SEQ ID NO: 36 | CMA-signal (QVELR) |
| SEQ ID NO: 37 | CMA-signal (QYFKS) |
| SEQ ID NO: 38 | CMA-signal (LKSFQ) |
| SEQ ID NO: 39 | CMA-signal (LKYFQ) |
| SEQ ID NO: 40 | CMA-signal (EKFLQ) |
| SEQ ID NO: 41 | CMA-signal (QREFK) |
| SEQ ID NO: 42 | CMA-signal (VDKFQ) |
| SEQ ID NO: 43 | SB100X transposase (AA) |
| SEQ ID NO: 44 | Frog Prince transposase (AA) |
| SEQ ID NO: 45 | Minos transposase (AA) |
| SEQ ID NO: 46 | ZB transposase (AA) |
| SEQ ID NO: 47 | Tdr1 transposase (AA) |
| SEQ ID NO: 48 | Passport transposase (AA) |
| SEQ ID NO: 49 | Linker sequence (KLGGGAPAVGGGPKAADK) |
| SEQ ID NO: 50 | NES-CMA-SB100X (AA) |
| SEQ ID NO: 51 | β-globin 5' UTR (NA) |
| SEQ ID NO: 52 | 5' UTR comprising an eIF4G aptamer (NA) |
| SEQ ID NO: 53 | β-globin 3'UTR (NA) |
| SEQ ID NO: 54 | α-globin 2 3'UTR (NA) |
| SEQ ID NO: 55 | 3'UTR comprising a WPRE (NA) |
| SEQ ID NO: 56 | βα-globin 3'UTR (NA) |
| SEQ ID NO: 57 | truncated β-globin 3'UTR (NA) |
| SEQ ID NO: 58 | SB100X (NA) |
| SEQ ID NO: 59 | NES-CMA-SB100X (NA) |
| SEQ ID NO: 60 | AA of NES combined with CMA-signal (LALKLAGLDIKFERQ) |
| SEQ ID NO: 61 | 5'UTR with a low predicted propensity to form a secondary structure |
| SEQ ID NO: 62 | A1K (NA) without poly(A)-tail |
| SEQ ID NO: 63 | A1H (NA) without poly(A)-tail |
| SEQ ID NO: 64 | A1I (NA) without poly(A)-tail |
| SEQ ID NO: 65 | A1O (NA) without poly(A)-tail |
| SEQ ID NO: 66 | A1P (NA) without poly(A)-tail |
| SEQ ID NO: 67 | B1M (NA) without poly(A)-tail |
| SEQ ID NO: 68 | B3M (NA) without poly(A)-tail |
| SEQ ID NO: 69 | A2M (NA) without poly(A)-tail |

### Figure Legends:

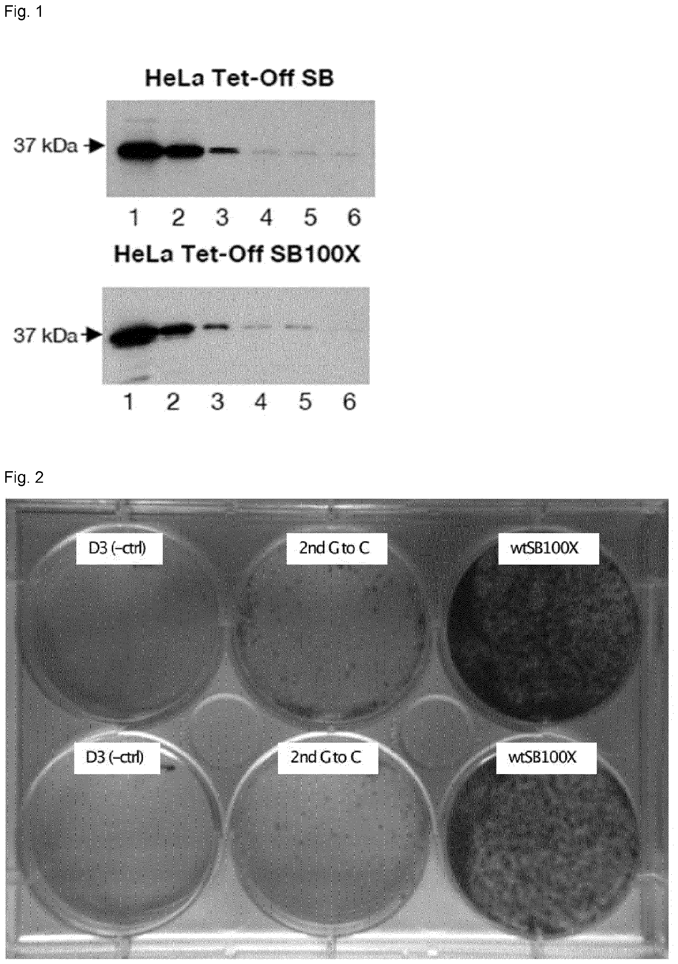

**Fig. 1****:** Western blot (polyclonal anti-SB) from HeLa Tet-Off SB or HeLa Tet-Off SB100X lines after different incubation times of doxycycline that shuts down transposase expression. 1) No doxycycline added. 2)-6) samples were taken after the cells were incubated in doxycycline for 1-4 days or for 6) one week. Both of the SB (wild type) and the SB100X transposase proteins persist for several days.
**Fig. 2****:** Targeting SB100X for ubiquitin-mediated proteasomal degradation based on the N-end-rule protein degradation pathway, *Strategy 1:* Changing the amino acid in the second position (N2) of the transposase to target the transposase for ubiquitin-proteasomal degradation results in compromised transpositional activity. A representative transposition assay in HeLa cells grown on puromycin-containing medium is shown. Cells were exposed to a plasmid expressing a *Sleeping Beauty* transposon comprising a puromycin resistance gene and SB100X transposase harbouring a G (Glycine) to C (Cysteine) modification at the 2^{nd} N-terminal amino acid position. Left: negative control D3 (catalytically inactive mutant transposase d3SB), Middle: transposase harbouring a G to C modification at N-terminal amino acid position 2, Right: positive control (wild type SB100X). Experiment was performed in duplicates.

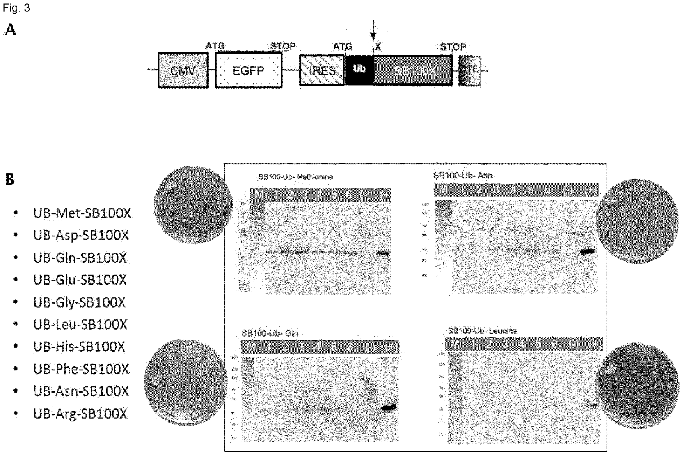

**Fig. 3****:** Targeting SB100X for ubiquitin-mediated proteasomal degradation based on the N-end-rule protein degradation pathway. *Strategy 2:* **(A)** SB100X gene was fused downstream to human ubiquitin in a strategy based on the N-end-rule protein degradation pathway (Mulder et al., 2000). A similar strategy was used to target HIV-1 integrase to degrade. The downward arrow denotes the location of the post-translational cleavage between ubiquitin and SB100X **(B)** A series of SB100X variants differing in the amino acid at position 1 was generated, transfected to HeLa cells and tested for both transposition activity and protein stability (cycloheximide assay, 1-6 hours); (-) not transfected; (+) not treated. Only mutants with comparably high transpositional activity are shown. However, none of the mutants tested exhibited a reduced half-life.

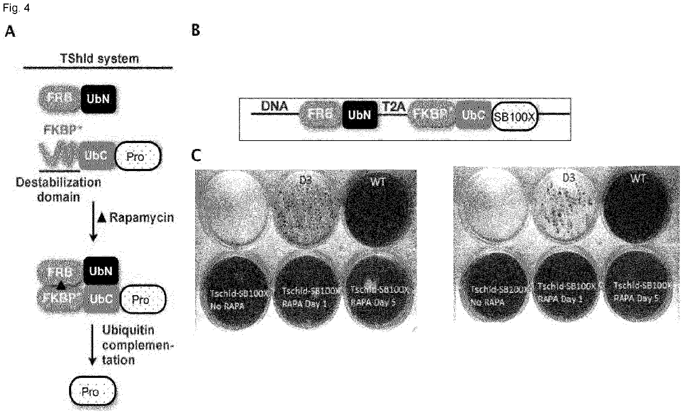

**Fig. 4****:** Targeting SB100X for ubiquitin-mediated proteasomal degradation based on the N-end-rule protein degradation pathway. *Strategy 3:* Use of a Shld1-dependent destabilization domain as described in Banaszynski et al., 2006 for targeting SB100X to the ubiquitin-mediated proteasomal degradation pathway. The system is based on a modified FKBP (*) that provides a tightly controllable heterodimerisation of FKBP*/FRB in the presence of Rapamycin. Fused to the Ubi subunits (UbN, UbC), the proteasomal degradation of the protein of interest (Pro) can be controlled. **(A)** Schematic overview of the TShld system as described in Banaszynski et al., 2006. **(B)** Schematic overview of a modified version of the system according to **(A)** for targeting SB100X for proteasomal degradation. **(C)** Two representative colony formation - transposition assays. Upper: Left: no transposase, Middle: catalytically inactive mutant transposase D3SB (D3), Right: wild type SB100X (WT) Lower: TShld-SB100X in the presence of the ligand, rapamycin (RAPA). Left: no Rapa, Middle RAPA 1 day, Right: RAPA 5 day. One would expect a controllable degradation of SB100X, however no difference was observed.

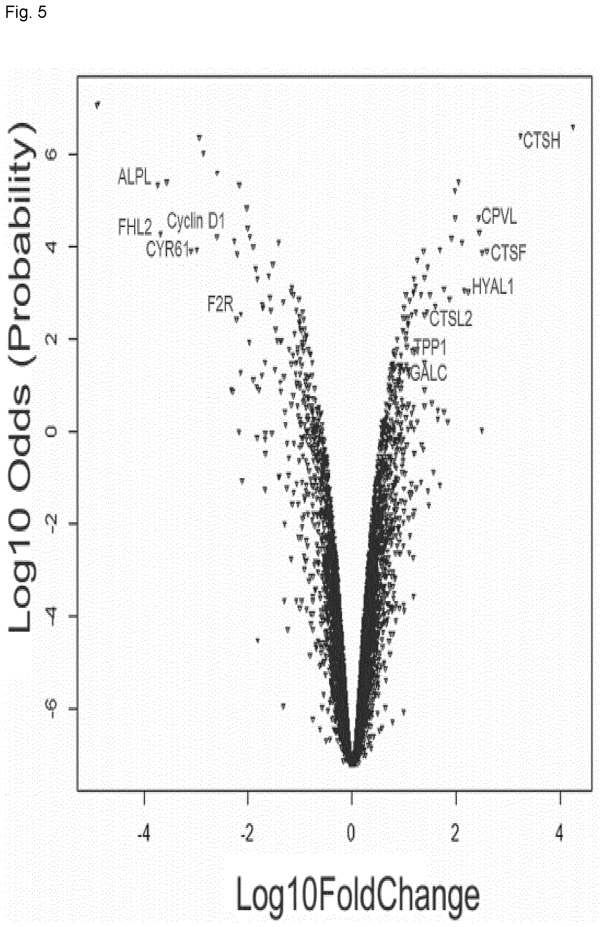

**Fig. 5****:** High throughput transcriptome analysis suggests that Sleeping Beauty-mediated transposition is associated with an upregulated expression of several cathepsins, known to be important in overall degradation of lysosomal proteins. Volcano plot displays significantly dysregulated genes in response to the expression of the SB100X protein in Hela cells. Left: downregulated, Right: upregulated. Note that the upregulated genes belong to the gene ontology of Lysosomal degradation (10⁻⁴).

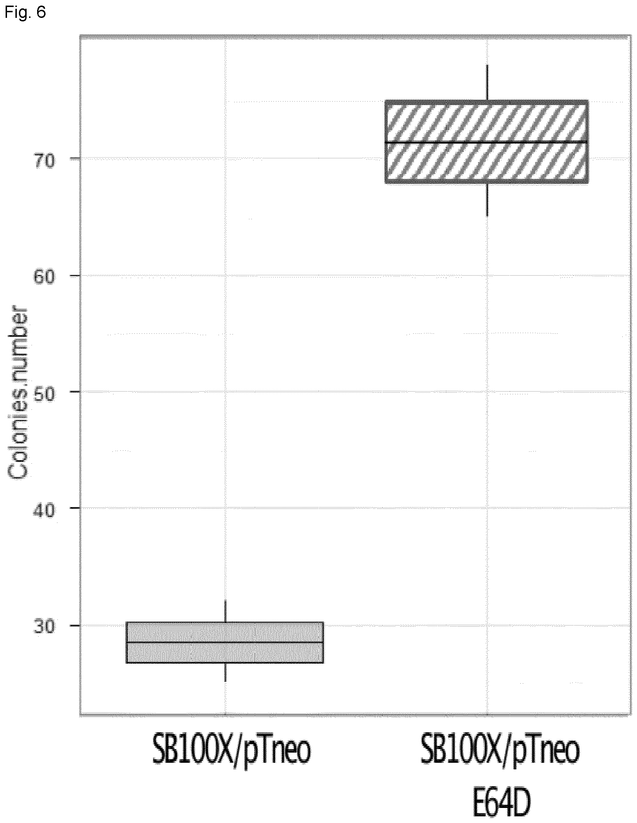

**Fig. 6****:** Inhibition of the lysosomal pathway enhances SB-mediated transposition. Graph depicts the quantification of a colony formation - transposition assay in the presence of the B- and L-cathepsin inhibitor E64D.

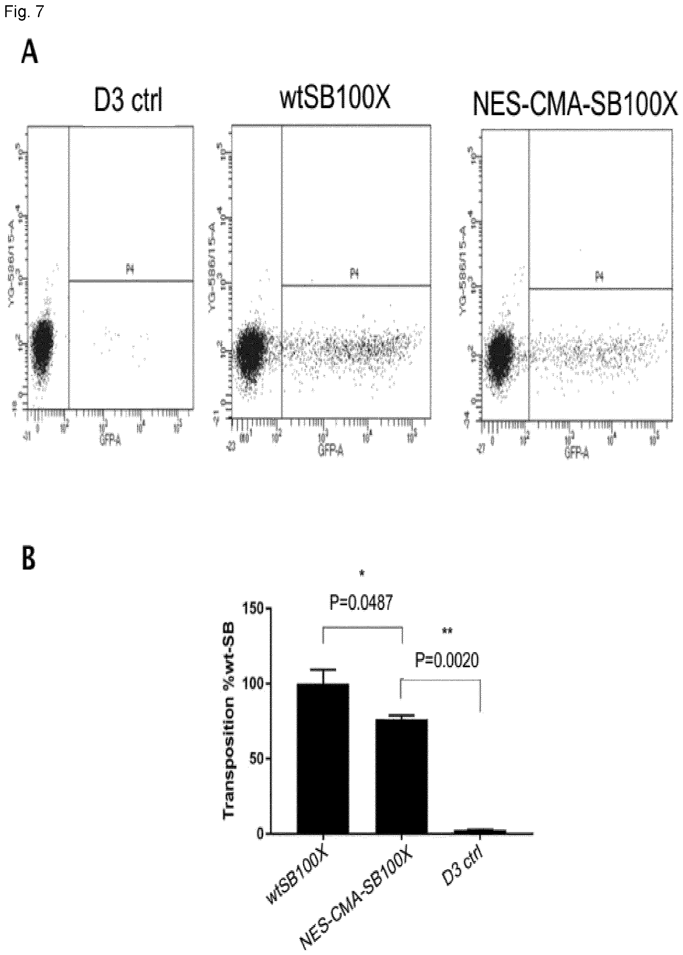

**Fig. 7****:** The NES-CMA-SB100X variant exhibits a modestly decreased transpositional activity compared to wild type (wt)SB100X in an EGFP-based transposon assay. **(A)** FACS sorting of GFP-expressing HeLa cells after SB-mediated transposition. The NES-CMA-SB100X variant was compared to the wtSB100X and the catalytically inactive mutant transposase (d3SB, negative control). The reporter plasmid was co-transfected with the constructs encoding the SB transposase variants. The assay is based on the integration of the SB transposon marked by the EGFP reporter sequence. Upon integration, the measured GFP fluorescent signal reports on the catalytic activity of the transposase. **(B)** Quantification of the results of (A).

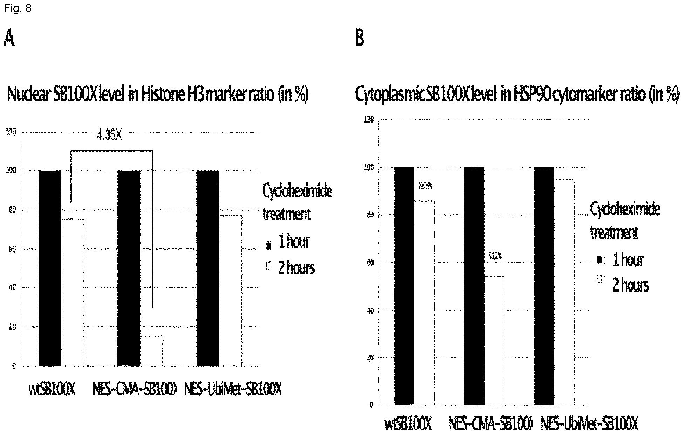

**Fig. 8****:** NES-CMA-SB100X variant exhibits significantly faster degradation compared to the wild type (wt)SB100X transposase and the NES-UbiMet-SB100X control variant **(A-B)** To determine the half-life of the NES-CMA-SB100X variant, 500.000 HeLa cells were transfected using 450 ng plasmid constructs encoding either wtSB100X (wild type), the NES-CMA-SB100X (targeted for lysosomal degradation) or the NES-UbiMet-SB100X (targeted for ubiquitin-mediated degradation) variants. Two days post-transfection, the medium of the cells was changed to a fresh medium, supplemented with 100 mM cycloheximide to inhibit *de novo* protein production. Following the incubation with cycloheximide (1 and 2 hours), the cells were collected and fractionated, and the different transposase protein variants were quantified by densitometry, based on the Western blot. The amount of the transposase protein molecules was normalized to the cell-fraction marker proteins. Histone H3 was used as nuclear fraction marker protein whereas HSP90 served as a marker for the cytosolic fraction. **(A)** Quantification of nuclear localization of the indicated SB100X variants in relation to histone H3 after 1 or 2 hour exposure to 100 mM cycloheximide. Note that the NES-CMA-SB100X transposase showed 4,36x faster degradation compared to wild type SB100X. **(B)** Quantification of cytoplasmic localization of the indicated SB100X variants in relation to HSP90 after 1- or 2-hour exposure to 100 mM cycloheximide. Note that the level of the SB transposase protein, following 1h cycloheximide treatment was set as 100%. The depletion of total SB protein between time points of 1 and 2 h cycloheximide treatment was: SB100X - 35%, NES-CMA-SB00X -75,4%.

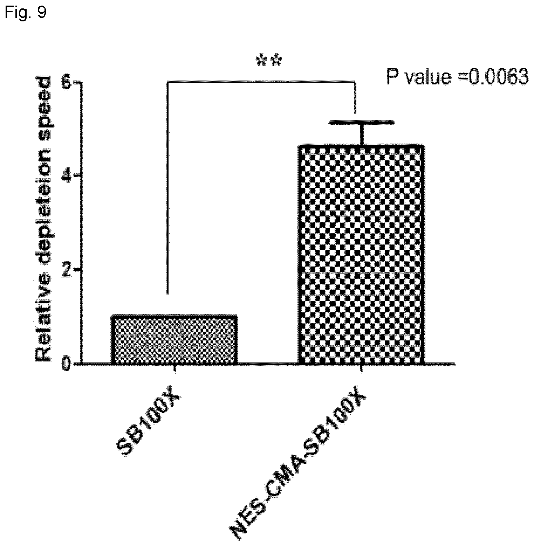

**Fig. 9****:** NES-CMA-SB100X variant exhibits significantly faster degradation compared to the wild type (wt)SB1 00X transposase (measured between time points of 1 and 24 hour treatment with cycloheximide). Three independent experiments. The SB transposase levels were determined from the whole cellular lysate (no fractionation). The amount of each transposase variant was quantified by densitometry and their quantities were normalised by the total protein amount using the Chemi Doc^{™} MP Imaging system (BioRad).

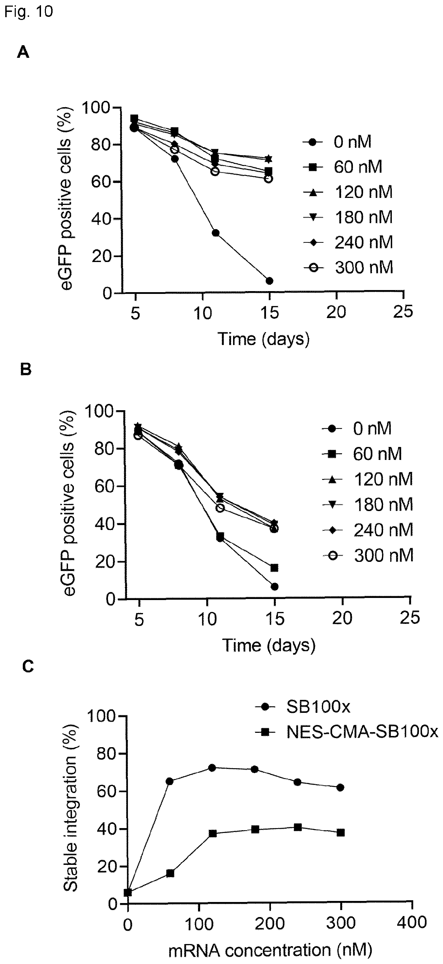

**Fig. 10****:** Transpositional activity of NES-CMA-SB100X expressed from in vitro-transcribed (ivt) mRNA. Ju76 cells were transfected with 50 µg/mL pT4-MP71-GFP plasmid DNA and 30-150 µg/mL ivt mRNA encoding either wild type SB100X **(A)** or the NES-CMA-SB100X variant **(B).** Percentage of eGFP positive cells were followed for 15 days to measure stable integration. **(C)** Graph depicts correlation between integration efficiency as seen by stable expression of eGFP and mRNA concentration for the tested SB100X variants.

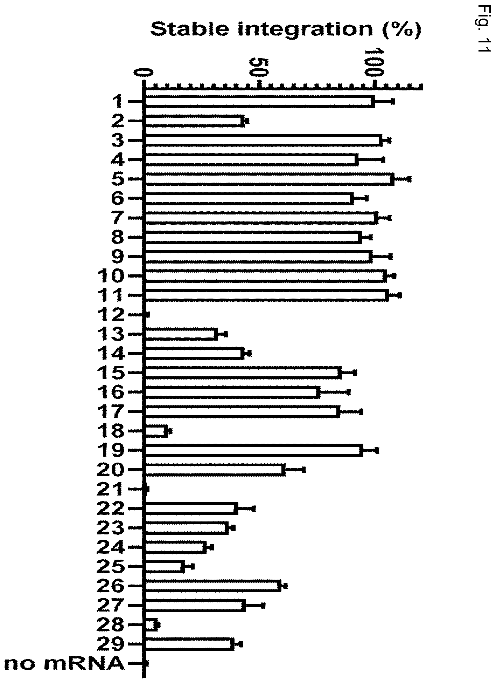

**Fig. 11****:** Stable gene integration in Jurkat cells with modified CMA-NES-SB100x constructs. Results were normalized to SB100x mRNA (construct 1). 1: SB100X (SEQ ID NO: 1); 2: NES-CMA-SB100X (SEQ ID NO: 2); 3: SEQ ID NO: 3; 4: SEQ ID NO: 4; 5: SEQ ID NO: 5; 6:SEQ ID NO: 6; 7: SEQ ID NO: 7; 8: SEQ ID NO: 8; 9: SEQ ID NO: 9; 10: SEQ ID NO: 10; 11: SEQ ID NO: 11; 12: SEQ ID NO: 12; 13: SEQ ID NO: 13; 14: SEQ ID NO: 14; 15: A2M_U1 (SEQ ID NO: 5, Pseudo-UTP substituted); 16: A2M_U2 (SEQ ID NO: 5, N1-Methylpseudo-UTP substituted); 17: A2M_U3 (SEQ ID NO: 5, 5-Methoxy-UTP substituted); 18: A2M_U4 (SEQ ID NO: 5, 2-Thio-UTP substituted); 19: A2M_C1 (SEQ ID NO: 5, 5-Methyl-CTP substituted); 20: A2M_C2 (SEQ ID NO: 5, N4-Acetyl-CTP substituted); 21: A2M_A1 (SEQ ID NO: 5, N6-Methyl-ATP substituted); 22: SEQ ID NO: 22; 23: SEQ ID NO: 23; 24: SEQ ID NO: 24; 25: SEQ ID NO: 25; 26: SEQ ID NO: 26; 27: SEQ ID NO: 27; 28: SEQ ID NO: 28; 29: SEQ ID NO: 29. The used capping structure was either a CapO structure (RNA constructs 1 and 2), or Cap1 to increase translational efficiency and stability (RNA constructs 3-29). Either no 5'UTR (RNA constructs 1 and 2), the 5'UTR of the human β-globin gene (RNA constructs 3-9, 15-29), an elF4G aptamer (RNA constructs 10 and 11), a 5'UTR comprising the J-K stem-loop region of the encephalomyocarditis virus IRES (RNA constructs 12) or a 5'UTR with no or weak predicted secondary structural elements such as hairpins (RNA constructs 13 and 14) was used. Either no CMA motif coupled to a NES (RNA constructs 1 and 3), KFERQ (RNA constructs 2, 4-21), VKKDQ (RNA construct 22), KFFEQ (RNA construct 23), KIREI (RNA construct 24), QVELR (RNA construct 25), QYFKS (RNA construct 26), LKSFQ (RNA construct 27) LKYFQ (RNA construct 28) or EKFLQ (RNA construct 29) was used. Either a CDS of SB100x (RNA constructs 1 and 3) or a CDS of CMA-NES-SB100x (RNA constructs 2, 4-29) was used. Either no 3'UTR (RNA constructs 1 and 2), the 3' UTR of human β-globin (RNA constructs 3 and 6), the 3'UTR of WPRE (RNA constructs 4, 12-14), two 3'UTRs of human β-globin directly after another (RNA constructs 5, 10-11, 15-29), the 3'UTR of human α-globin (RNA construct 7), a combination of human β-globin and α-globin 3'UTR (RNA construct 8) or a shortened version of the 3'UTR of human β-globin (RNA construct 9) was used. Either an enzymatically added poly(A) tail of 100-300 nucleotides (RNA constructs 1 and 2), or a transcribed poly(A)-tail of 90 nucleotides (RNA constructs 3-29) was used. Either non-modified nucleotides (RNA constructs 1-14, 22-29), UTP fully replaced with pseudo-UTP (RNA construct 15, A2M_U1), N1-methylpseudo-UTP (RNA construct 16, A2M_U2), 5-Methoxy-UTP (RNA construct 17, A2M_U3) or 2-Thio-UTP (RNA construct 18, A2M_U4), CTP fully replaced with 5-Methyl-CTP (RNA construct 19, A2M_C1) or N4-Acetyl-CTP (RNA construct 20, A2M_C2) or ATP fully replaced with N6-Methyl-ATP (RNA construct 21, A2M_A1) were used.

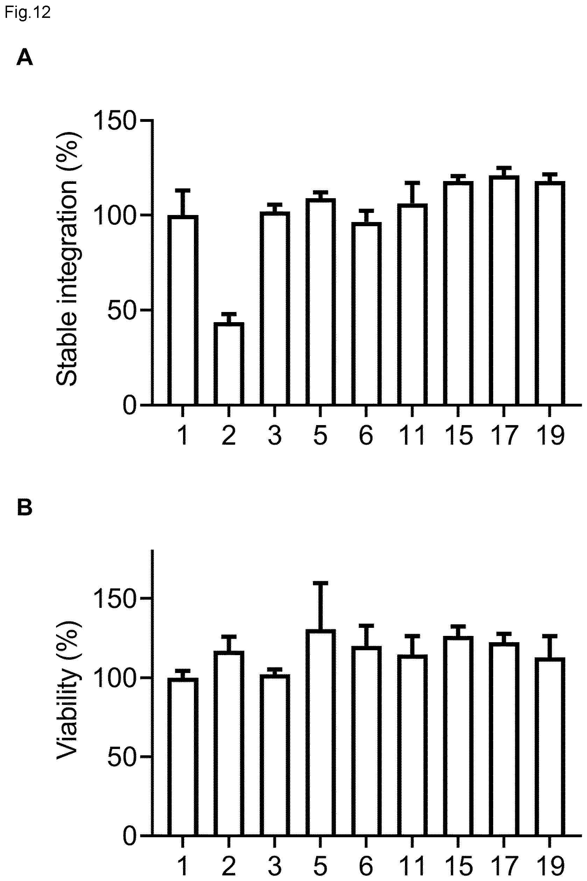

**Fig. 12****:** Several variants were tested as mRNA in primary human CD3+ cells for **(A)** pT4-mediated stable integration of a reporter gene and **(B)** cell viability. **(A)** Stable integration of a reporter gene into human CD3+ cells 15 days after transfection. **(B)** Cell viability 15 days after transfection. 1: SB100X (SEQ ID NO: 1); 2: NES-CMA-SB100X (SEQ ID NO: 2); 3: SEQ ID NO: 3; 5: SEQ ID NO: 5; 6: SEQ ID NO: 6; 11: SEQ ID NO: 11; 15: A2M_U1 (SEQ ID NO: 5, Pseudo-UTP substituted); 17: A2M_U3 (SEQ ID NO: 5, 5-Methoxy-UTP substituted); 19: A2M_C1 (SEQ ID NO: 5, 5-Methyl-CTP substituted).

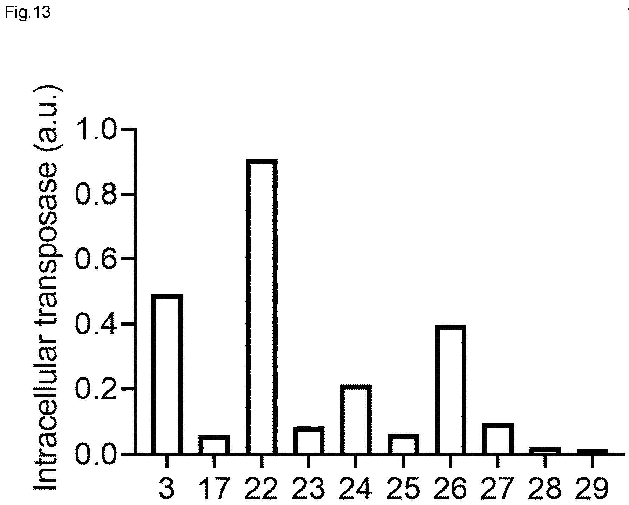

**Fig. 13****:** Intracellular concentration of Sleeping Beauty transposase of constructs with different CMA motifs four days after transfection as determined by Western Blot. Amounts are normalized to intracellular transposase concentrations four hours after transfection. 3: SEQ ID NO: 3; 17: A2M_U3 (SEQ ID NO: 5, 5-Methoxy-UTP substituted); 22: SEQ ID NO: 22; 23: SEQ ID NO: 23; 24: SEQ ID NO: 24; 25: SEQ ID NO: 25; 26: SEQ ID NO: 26; 27: SEQ ID NO: 27; 28: SEQ ID NO: 28; 29: SEQ ID NO: 29.

### Examples

### Example 1

### Improving the SB100X transposon system efficacy and safety: Construction of a Short Half-life Version of the Hyperactive Sleeping Beauty (SB) Transposase SB100X

### Introduction

Improving the biosafety of cell engineering systems has gained attention from regulatory authorities. The gene delivery vector should be removed from the cells to be modified as soon as possible to avoid unwanted gene toxicity.

The integrating non-viral *Sleeping Beauty* (SB) transposon system is a widely used genomic engineering tool and is routinely used in wide range of cell types [Ivics et al., 1997, Izsvak et al., 2000]. The hyperactive transposase variant (SB100X) was demonstrated to integrate transgenes into various human cell types with high efficiency [Mates et al., 2009]. Currently, the SB100X system is a popular therapeutic delivery and expression vector in humans (reviewed in [Kebriaei et al., 2017. Narayanavari et al., 2017, Sandoval-Villegas et al., 2021]). There are continuous efforts to improve the biosafety of the system.

The *Sleeping Beauty* (SB) transposon system consists of two components. The transposase is a recombinase enzyme that catalyzes the transposition of the SB transposon. Following expression, the transposase catalyzes the transposition reaction immediately, and its presence is not required any longer. In fact, the continued presence of the transposase is a safety problem, since the transposase can trigger re-mobilization events, leading to genomic instability. The SB100X transposase is predicted to have a relatively long half-life (>30 hours) [Voigt et al., 2016]. After induction, it is possible to detect transposition activity for several days (Figure 1A and B). In this time span, the transposase might support multiple runs of unwanted transposition. In addition, the transposase is sensitive to aggregation, and the persisting transposase molecules can contribute to nuclear aggregates, causing cellular toxicity, especially when the transposase is overdosed. Notably, using currently available mammalian cloning vectors, the expressed proteins are frequently overexpressed.

The aim of this study was to reduce the lifetime of the transposase protein, thereby eliminating toxicity problems, and improve biosafety of the system.

In the prior art, the half-life of the transposase was not altered. We tested the concept that it might be possible to direct the SB100X transposase into a protein degradation pathway by fusing a degradation signal to the SB100X transposase.

In eukaryotic cells, damaged proteins or organelles can be cleared by proteasomes or lysosomes [de Duve, 2005, Dikic, 2017]. The two pathways are independent but interconnected with each other [Wang et al., 2014].

### Results

The SB100X transposase is a relatively stable protein and it can be detected for several days in the cells [Mates et al., 2009] and Fig. 1. To improve the safety of the SB100X transposon system for clinical application, the present inventors targeted the *Sleeping Beauty* transposase to either the (1) ubiquitin-mediated proteosomal (UPS) or the (2) autophagy-lysosomal (ALP) degradation pathways:
The ubiquitin-proteasome system (UPS) is one of the major route through which eukaryotic cells achieve selective protein degradation [Kleiger et al., 2014]. The UPS is the primary proteolytic route for small, short-lived, soluble, misfolded, and damaged proteins [reviewed in Dikic, 2010]. Notably, targeting proteins to the ubiquitin degradation pathway has been successfully applied for a number of proteins [Zhao et al, 2022]. The first degrons to be discovered were located at the N-terminus of proteins [Bachmair et al., 1986]. The ALP recognizes and removes long-lived, large, and potentially dangerous cellular components such as insoluble protein aggregates and dysfunctional or superfluous organelles [reviewed in Dikic, 2010]. The ALP process delivers cytosolic constituents to lysosomes for their subsequent degradation.

Notably, UPS and ALP intersect and communicate at multiple points [Wang and Robbins 2014], influence each other, and mutually control the levels of their key components to coordinate and balance their actions in proteostasis. In chaperon-mediated autophagy (CMA), proteins are selected by chaperones and targeted to lysosomes, where the complex is translocated across the lysosome membrane for degradation.

As the SB transposase is relatively small protein, we first tried to target it to the UPS (1a-c).

### 1a. Changing the amino acid in the second position (N2) of the transposase

The N-terminal residue influences protein stability through the *N-end rule* pathway. Computational identification of destabilizing N-terminal motifs (degrons) in protein revealed that the majority of the top 100 predicted destabilizing N-terminal motifs encoded either lysine (K2), arginine (R2) or cysteine (C2) at the second position [Timms et al., 2019]. Therefore, we exchanged the 2^{nd} position of the SB transposase from G (Glycin) to C (Cystein). However, changing the amino acid in the second position (G to C) of the SB transposase, resulted in a compromised activity (Fig. 2).

### 1b. Strategy to facilitate protein degradation by targeting the transposase to the proteosome degradation pathway

The HIV-1 integrase and the SB transposase are enzymatically related [Voigt et al., 2016]. It has been shown that the HIV-1 integrase was a physiological substrate of the *N-end rule degradation* pathway [Mulder et al., 2000]. By changing the N-terminal residue of the HIV-1 integrase it was possible to generate various HIV-1 versions with different stability [Mulder et al., 2000].

In this approach, following the HIV-1 example [Mulder et al., 2000], the SB100X gene was fused downstream to the human ubiquitin (Figure 3). A series of SB100X variants differing in the amino acid sequence in position 1 were generated and tested for both transposition activity and protein stability (Figure 3). The *downward arrows* denote the location of the post-translational cleavage between ubiquitin and X-transposase (SB100X) (Fig. 3).

**1c.** The third approach following the strategy suggested by [Banaszynski et al., 2006] was not successful either (Fig. 4). This strategy provides a sensitive regulation of stability to proteins targetable to the Ubiquitin-mediated degradation pathway.

Collectively (1a-c), the cycloheximide protein stability assays have revealed that none of the generated mutations affected the half-life of the SB100X transposase, suggesting that the SB100X transposase cannot be targeted to the Ubiquitin-mediated, UPS degradation pathway per se.

### 2. Strategy to facilitate protein degradation by targeting the transposase to the autophagy-mediated protein degradation pathway

To investigate cellular responses to the presence of the SB transposase, we performed a high throughput transcriptome analysis. This approach revealed that SB transposition upregulated the expression of several cathepsins (Fig. 5), known to be important in overall degradation of lysosomal proteins, and participate in autophagy (reviewed in Yadati et al, 2020). Notably, inhibiting the lysosomal pathway using the cathepsin (B, L) inhibitor E64D enhanced SB-mediated transposition (Fig. 6). Collectively, these observations suggested that the SB100X transposase is sensitive to the 'Lysosomal' protein degradation pathway.

Following the failure of the previous attempts (proteosomal targeting strategies 1a-c), we thus decided to try an alternative approach, in which we target the transposase to the chaperone-mediated autophagy (CMA) protein degradation pathway, utilized by a selective subset of proteins. Notably, not seen by other targeted protein degradation strategies, this approach is less established and faces several unsolved challenges (reviewed in Zhao et al, 2022).

The targeting strategy based on a specific CMA signal that is a unique pentapeptide (KFERQ) [Dice, 1990; Wing et al., 1991]. In CMA, the heat shock 70 (HSC70) chaperon recognizes protein substrates with the KFERQ motif [Dice, 1990; Wing et al., 1991]. The HSC70-KFERQ-protein substrate complex then binds to lysosomal associated membrane protein 2A (LAMP2), and is translocated to the lysosome lumen for degradation [Gough et al 1995] . Thus, in principle, a protein containing the KFERQ motif could be exploited to degrade a protein, representing an alternative approach in targeted protein degradation [Fan et al, 2014].

To target the SB100X by CMA, we fused the transposase with a specific autophagy-mediated protein degradation signal, a unique pentapeptide (KFERQ) [Dice, 1990; Wing et al., 1991]. Furthermore, as the SB100X transposase is a nuclear protein, we hypothesised that exporting the expressed SB100X protein from the nucleus in combination with the targeting strategy will facilitate the degradation kinetic of the SB100X transposase. Therefore, to facilitate the SB100X transposase protein clearance from the nuclear space, the pentapeptide signal was additionally fused N-terminally with a nuclear export signal (NES) of protein kinase inhibitor-α (LALKLAGLDIKFERQ, SEQ ID NO: 60).

The position of the NES: The functionally of the NES signal is independent of its position [https://doi.org/10.1016/C2010-0-64974-1] within the protein sequence, however the SB transposase is sensitive for modifications that could compromise activity and/or structural instability [Narayanavari et al, 2017]. The SB transposase can tolerate N-terminal tagging, whereas C-terminal tagging compromises activity. In principle, embedding the targeting signal(s) in the protein is possible, but not obvious and needs careful design and optimisation. Furthermore, it was important to use a linker between the transposase and the NES-CMA signals. Thus, the binary signal sequence was joined to the SB100X transposase using a linker peptide sequence (KLGGGAPAVGGGPKAADK, SEQ ID NO: 49) published elsewhere [Kovac et al., 2020].

The activity of the new NES-CMA-SB100X variant was measured using the EGFP-based transposon assay in HeLa cells. This assay is based on the integration of the SB transposon marked by the EGFP reporter sequence. Upon integration the measured GFP fluorescent signal reports on the catalytic activity of the transposase. Here, the NES-CMA-SB100X variant was compared to the wtSB100X and the catalytically inactive mutant transposase (d3SB, negative control) in human HeLa cells (Fig. 7). The reporter plasmid was co-transfected with the constructs encoding the SB transposase variants. Note that at day 2 post-transfection, the GFP signal is used to determine the transfection efficiency (TE, %). At 14 days post-transfection, the background from the non-integrated background is barely detectable, and the GFP signal is attributed to the integrated GFP marked transposon (Int). The transposition efficacy of the transposase was then normalized by TE (i.e., Int/TE). The "activity of the NES-CMA-SB100X transposase version" is therefore Int/TE as a percentage of wtSB100X. The NES-CMA-SB100X has -70% the activity of the wtSB100X (Fig. 7).

To determine the half-life of the NES-CMA-SB100X variant, 500.000 HeLa cells were transfected using 450 ng plasmid constructs encoding the SB variants. To ensure comparable expressions, the SB transposase genes were under the control of CMV promoter in all of the constructs. The tested transposase versions were wtSB100X, NES-CMA-SB100X and NES-UbiMet-SB100X. NES-CMA-SB100X and NES-UbiMet-SB100X are transposase versions both possessing the NES signal, but targeted to the CMA or the Ubiquitin-mediated degradation pathways, respectively. Two days post-transfection, the medium of the cells was changed to a fresh medium, supplemented with 30-100 mM cycloheximide (inhibits *de novo* protein production). Following the incubation with cycloheximide (1 and 2 hours), the cells were collected and fractionated using the NE-PER kit (Thermo Scientific^{™}), following manufacturer's recommendations. The cell extracts were loaded on SDS-PAGE, and the different transposase protein variants were quantified by densitometry. The amount of the transposase molecules was normalized to the cell-fraction marker proteins. For the nuclear fraction, we used the nuclear fraction marker protein, histone H3; for the cytosolic fraction, cytosolic marker protein; HSP90. This experiment clearly demonstrated that the combined NES-CMA signal was functional, and in the nuclear fraction the NES-CMA-SB100X version had an approximately 4,3X faster degradation when compared to the wtSB100X transposase (Fig. 8). Using the same experimental setup, but no fractionation, the total amount of the transposase versions was analysed by densitometry and their quantities were normalised by the total protein amount using by the Chemi Doc TM MP Imaging system (BioRad). In these experiments, the NES-CMA-SB100X transposase showed statistically significant (**) 5,4x faster degradation compared to the original SB100X (Fig. 9).

### 3. Strategy to increase transposition efficiency while maintaining a shorter SB100x half-life through mRNA modifications

mRNA encoding SB100x was transcribed in vitro and its efficiency tested in a transposition assay in Jurkat cells (Fig. 10A). Stable transposition is tracked by co-transfection of a plasmid carrying a fluorescent reporter gene (enhanced green fluorescent protein) flanked by pT4 transposon sequences. Over time, only stabile transposed cells show intracellular fluorescence measured by flow cytometry. Compared to NES-CMA-SB100x (Fig. 10B), wild-type SB100x performed approximately twice as good as NES-CMA-SB100x (Fig. 10C).

To increase the efficiency of NES-CMA-SB100x while retaining a short half-life, we made modifications to the capping structure of the transcribed mRNA, the 5' untranslated region (5'UTR), the coding sequence of NES-CMA-SB100x, the 3' untranslated region (3'UTR), the poly(A)-tail, included modified nucleosides and used additional CMA motifs. mRNA of all constructs was generated and tested at optimized concentrations in Jurkat cells (Fig. 11). The used capping structure was either a CapO structure (RNA constructs 1 and 2), or Cap1 to increase translational efficiency and stability (RNA constructs 3-29). We used either no 5'UTR (RNA constructs 1 and 2), the 5'UTR of the human β-globin gene (RNA constructs 3-9, 15-29), an elF4G aptamer (RNA constructs 10 and 11), a 5'UTR comprising the J-K stem-loop region of the encephalomyocarditis virus IRES (RNA construct 12) or a 5'UTR with no or weak predicted secondary structural elements such as hairpins (RNA constructs 13 and 14). We either use no CMA motif coupled to a NES (RNA constructs 1 and 3), KFERQ (RNA constructs 2, 4-21), VKKDQ (RNA construct 22), KFFEQ (RNA construct 23), KIREI (RNA construct 24), QVELR (RNA construct 25), QYFKS (RNA construct 26), LKSFQ (RNA construct 27) LKYFQ (RNA construct 28) or EKFLQ (RNA construct 29). We used either a CDS of SB100x (RNA constructs 1 and 3) or a CDS of CMA-NES-SB100x (RNA constructs 2, 4-29). We used either no 3'UTR (RNA constructs 1 and 2), the 3' UTR of human β-globin (RNA constructs 3 and 6), the 3'UTR of WPRE (RNA constructs 4, 12-14), two 3'UTRs of human β-globin directly after another (RNA constructs 5, 10-11, 15-29), the 3'UTR of human α-globin (RNA construct 7), a combination of human β-globin and α-globin 3'UTR (RNA construct 8), or a shortened version of the 3'UTR of human β-globin (RNA construct 9). We used either an enzymatically added poly(A) tail of 100-300 nucleotides (RNA constructs 1 and 2), or a transcribed poly(A)-tail of 90 nucleotides (RNA constructs 3-29). We used either non-modified nucleotides (RNA constructs 1-14, 22-29) or fully replaced UTP with pseudo-UTP (RNA construct 15), N1-methylpseudo-UTP (RNA construct 16), 5-Methoxy-UTP (RNA construct 17) or 2-Thio-UTP (RNA construct 18), CTP with 5-Methyl-CTP (RNA construct 19) or N4-Acetyl-CTP (RNA construct 20) or ATP with N6-Methyl-ATP (RNA construct 21).

In this first screening, several variants were identified that showed a similar or higher stable integration efficiency in Jurkat cells at optimized conditions than both wildtype SB100x and non-modified CMA-NES-SB100x. Several variants were tested in primary human CD3+ cells for pT4-mediated stable integration of a reporter gene (Fig. 12). RNA construct 17 performed best regarding relative integration efficiency, and significantly better than wild-type SB100x sequence regarding cell viability 15 days post transfection and expansion of the culture.

To ensure that the beneficial characteristics seen in Jurkat and CD3+ cells were not caused by an extended half-life of the CMA-NES-SB100x through the mRNA modifications, we measured the relative abundance of transposase 96 hours after transfection for several sequences (Fig. 13). As expected, most CMA-containing constructs (RNA constructs 17, 23-29) showed a significantly lower half-life than wild-type SB100x with similar mRNA modifications. Interestingly, the VKKDQ CMA motif - derived from the amyloid-beta precursor protein gene - in RNA constructs 22 derived from the amyloid-beta precursor protein gene increased the half-life of the transposase.

### Materials and Methods

### Cell culture and cell transfection

Human cervical carcinoma cells (HeLa) were cultured in Dubelcco's modified Eagle's medium (DMEM) with 4.5 g/l D-glucose containing 10% fetal bovine serum, supplemented with penicillin (100 µg/ml) and streptomycin (100 µg/ml) at 37°C and 5% CO₂. In general, the cells were seeded in 6-well plates and transfection was performed using JetPrimeTM or *Lipofectamine*^{®}*-3000* (Thermo Fischer) reagents and following the recommended manufacturer's protocol. Jurkat cells were cultured in RPMI medium supplemented with Glutamax, FBS, MEM, sodium pyruvate, penicillin (100 µg/ml) and streptomycin (100 µg/ml) at 37°C and 5% CO_{2.}.CD3+ cells were cultured in TexMACS medium (Miltenyi) supplemented with interleukins at 37°C and 5% CO₂. Cells were transfected on a 4D Nucleofector (Lonza).Cell cultures were analysed by flow cytometry on a MACSQuant (Miltenyi).

### In vitro transcribed mRNA

Plasmids encoding different (NES-CMA)-SB100x variants were linearized prior to transcription and, were necessary, enzymatically polyadenylated. All reagents were supplied by Jena Bioscience GmbH. mRNAs were purified with Mag-Bind TotalPure NGS beads (Omegabiotek) and eluted in water.

### Western blotting

Cells were collected from 6-well plates for Western blot analysis. The cells were subsequently washed with phosphate-buffered saline (PBS) and lysed on ice for 40 min in lysis buffer containing 50 mM TRIS HCI pH 8.0, 10 mM EDTA, 100 mM NaCl, 5% glycerol, 1% NP-40 and Protease Inhibitor Mini Tablets, EDTA Free (Pierce^{™}) and Benzonase Nuclease (NOVAGEN) as recommended by the manufacturer. Total lysates were resolved by 8-12 or 4-20% Mini-Protean^{®} TGX^{™} SDS-PAGE Gel and transferred to PVDF membranes by Bio-Rad Trans-Blot^{®}Turbo^{™} Transfer System. The membranes were blocked with TBS-T (TBS supplemented with 0.05% Tween-20) containing 5% non-fat dry milk and were incubated overnight at 4°C with primary antibodies (aSB (R&D Systems AF2798), aHSP90 (Cell Signaling #4874) and a Histone H3 (Cell Signaling #9715)) in appropriate dilutions in TBS-T containing 5% non-fat dry milk. Membranes were washed with TBS-T buffer, incubated for 1 hour at room temperature with Alkaline Phosphatase-conjugated (Sigma-Aldrich) or Horseradish Peroxidase-Conjugated (Promega) secondary antibodies in TBS-T containing 5% non-fat dry milk. The blots were subsequently washed with TBS-T. Bands were detected by ECLTM Prime Western Blotting Detection Reagent (Amersham) and images were then analysed by Chemi Doc^{™}MP Imaging System (Bio-Rad). Protein levels were determined by utilizing the densitometry analysis software of BioRad-ChemiDoc^{™} MP Imaging System.

### References

Wicker, T.; Sabot, F.; Hua-Van, A.; Bennetzen, J.L.; Capy, P.; Chalhoub, B.; Flavell, A.; Leroy, P.; Morgante, M.; Panaud, O.; et al. A unified classification system for eukaryotic transposable elements. Nat. Rev. Genet. 2007, 8, 973-982, doi:10.1038/nrg2165.
Ivics, Z.; Hackett, P.B.; Plasterk, R.H.; Izsvák, Z. Molecular Reconstruction of Sleeping Beauty, a Tc1-like Transposon from Fish, and Its Transposition in Human Cells. Cell 1997, 91, 501-510, doi:10.1016/S0092-8674(00)80436-5.
Amberger, M.; Ivics, Z. Latest Advances for the Sleeping Beauty Transposon System: 23 Years of Insomnia but Prettier than Ever: Refinement and Recent Innovations of the Sleeping Beauty Transposon System Enabling Novel, Nonviral Genetic Engineering Applications. Bioessays 2020, 42, e2000136, doi:10.1002/bies.202000136.
Mátés, L.; Chuah, M.K.L.; Belay, E.; Jerchow, B.; Manoj, N.; Acosta-Sanchez, A.; Grzela, D.P.; Schmitt, A.; Becker, K.; Matrai, J.; et al. Molecular evolution of a novel hyperactive Sleeping Beauty transposase enables robust stable gene transfer in vertebrates. Nat. Genet. 2009, 41, 753-761, doi:10.1038/ng.343.
Izsvák, Z.; Khare, D.; Behlke, J.; Heinemann, U.; Plasterk, R.H.; Ivics, Z. Involvement of a bifunctional, paired-like DNA-binding domain and a transpositional enhancer in Sleeping Beauty transposition. J. Biol. Chem. 2002, 277, 34581-34588, doi:10.1074/jbc.M204001200. Montaño, S.P.; Rice, P.A. Moving DNA around: DNA transposition and retroviral integration. Curr. Opin. Struct. Biol. 2011, 21, 370-378, doi:10.1016/j.sbi.2011.03.004.
Yang, W.; Lee, J.Y.; Nowotny, M. Making and breaking nucleic acids: two-Mg2+-ion catalysis and substrate specificity. Molecular Cell 2006, 22, 5-13, doi:10.1016/j.molcel.2006.03.013. WO2009/003671 European patent application 22 200 543.1 PCT/EP2022/075007
Querques, I.; Mades, A.; Zuliani, C.; Miskey, C.; Alb, M.; Grueso, E.; Machwirth, M.; Rausch, T.; Einsele, H.; Ivics, Z.; et al. A highly soluble Sleeping Beauty transposase improves control of gene insertion. Nat. Biotechnol. 2019, 37, 1502-1512, doi:10.1038/s41587-019-0291-z. EP3673053A
Kutay U, Güttinger S. Leucine-rich nuclear-export signals: born to be weak. Trends Cell Biol. 2005 Mar;15(3):121-4. doi: 10.1016/j.tcb.2005.01.005. PMID: 15752974.
Xu D, Farmer A, Collett G, Grishin NV, Chook YM. Sequence and structural analyses of nuclear export signals in the NESdb database. Mol Biol Cell. 2012 Sep;23(18):3677-93. doi: 10.1091/mbc.E12-01-0046. Epub 2012 Jul 25. PMID: 22833565; PMCID: PMC3442415. Kosugi S, Hasebe M, Tomita M, Yanagawa H. Nuclear export signal consensus sequences defined using a localization-based yeast selection system. Traffic. 2008 Dec;9(12):2053-62. doi: 10.1111/j.1600-0854.2008.00825.x. Epub 2008 Sep 25. PMID: 18817528.
Güttler T, Madl T, Neumann P, Deichsel D, Corsini L, Monecke T, Ficner R, Sattler M, Görlich D. NES consensus redefined by structures of PKI-type and Rev-type nuclear export signals bound to CRM1. Nat Struct Mol Biol. 2010 Nov;17(11):1367-76. doi: 10.1038/nsmb.1931. Epub 2010 Oct 24. PMID: 20972448.
Güttler T, Görlich D. Ran-dependent nuclear export mediators: a structural perspective. EMBO J. 2011 Aug 31;30(17):3457-74. doi: 10.1038/emboj.2011.287. PMID: 21878989; PMCID: PMC3181476.
Orenstein SJ, Cuervo AM. Chaperone-mediated autophagy: molecular mechanisms and physiological relevance. Semin Cell Dev Biol. 2010 Sep;21(7):719-26. doi: 10.1016/j.semcdb.2010.02.005. Epub 2010 Feb 20. PMID: 20176123; PMCID: PMC2914824. Miyakawa S, Oguro A, Ohtsu T, Imataka H, Sonenberg N, Nakamura Y. RNA aptamers to mammalian initiation factor 4G inhibit cap-dependent translation by blocking the formation of initiation factor complexes. RNA. 2006 Oct;12(10):1825-34. doi: 10.1261/rna.2169406. Epub 2006 Aug 29. PMID: 16940549; PMCID: PMC1581983.Remington's Pharmaceutical Sciences, 17th ed., 1985, Mack Publishing Co.
Terenin IM, Andreev DE, Dmitriev SE, Shatsky IN. A novel mechanism of eukaryotic translation initiation that is neither m7G-cap-, nor IRES-dependent. Nucleic Acids Res. 2013 Feb 1;41(3):1807-16. doi: 10.1093/nar/gks1282. Epub 2012 Dec 24. PMID: 23268449; PMCID: PMC3561988.
Izsvak Z, Ivics Z, Plasterk RH. Sleeping Beauty, a wide host-range transposon vector for genetic transformation in vertebrates. J Mol Biol. 2000;302(1):93-102. Epub 2000/08/31. doi: 10.1006/jmbi.2000.4047. PubMed PMID: 10964563.
Kebriaei P, Izsvak Z, Narayanavari SA, Singh H, Ivics Z. Gene Therapy with the Sleeping Beauty Transposon System. Trends Genet. 2017;33(11):852-70. Epub 20170927. doi: 10.1016/j.tig.2017.08.008. PubMed PMID: 28964527.
Narayanavari SA, Chilkunda SS, Ivics Z, Izsvak Z. Sleeping Beauty transposition: from biology to applications. Critical reviews in biochemistry and molecular biology. 2017;52(1):18-44. Epub 20161004. doi: 10.1080/10409238.2016.1237935. PubMed PMID: 27696897.
Sandoval-Villegas N, Nurieva W, Amberger M, Ivics Z. Contemporary Transposon Tools: A Review and Guide through Mechanisms and Applications of Sleeping Beauty, piggyBac and Tol2 for Genome Engineering. Int J Mol Sci. 2021;22(10). Epub 20210511. doi: 10.3390/ijms22105084. PubMed PMID: 34064900; PubMed Central PMCID: PMCPMC8151067.
Voigt F, Wiedemann L, Zuliani C, Querques I, Sebe A, Mates L, et al. Sleeping Beauty transposase structure allows rational design of hyperactive variants for genetic engineering. Nat Commun. 2016;7:11126. doi: 10.1038/ncomms11126. PubMed PMID: 27025571.
de Duve C. The lysosome turns fifty. Nat Cell Biol. 2005;7(9):847-9. doi: 10.1038/ncb0905-847. PubMed PMID: 16136179.
Dikic I. Proteasomal and Autophagic Degradation Systems. Annu Rev Biochem. 2017;86:193-224. Epub 20170501. doi: 10.1146/annurev-biochem-061516-044908. PubMed PMID: 28460188.
Wang X, Robbins J. Proteasomal and lysosomal protein degradation and heart disease. J Mol Cell Cardiol. 2014;71:16-24. Epub 20131114. doi: 10.1016/j.yjmcc.2013.11.006. PubMed PMID: 24239609; PubMed Central PMCID: PMCPMC4011941.
Kleiger G, Mayor T. Perilous journey: a tour of the ubiquitin-proteasome system. Trends Cell Biol. 2014;24(6):352-9. Epub 20140120. doi: 10.1016/j.tcb.2013.12.003. PubMed PMID: 24457024; PubMed Central PMCID: PMCPMC4037451.
Bachmair A, Finley D, Varshavsky A. In vivo half-life of a protein is a function of its amino-terminal residue. Science. 1986;234(4773):179-86. doi: 10.1126/science.3018930. PubMed PMID: 3018930.
Timms RT, Zhang Z, Rhee DY, Harper JW, Koren I, Elledge SJ. A glycine-specific N-degron pathway mediates the quality control of protein N-myristoylation. Science. 2019;365(6448). doi: 10.1126/science.aaw4912. PubMed PMID: 31273098; PubMed Central PMCID: PMCPMC7090375.
Mulder LC, Muesing MA. Degradation of HIV-1 integrase by the N-end rule pathway. J Biol Chem. 2000;275(38):29749-53. doi: 10.1074/jbc.M004670200. PubMed PMID: 10893419. Zhao L, Zhao J, Zhong K, Tong A, Jia D. Targeted protein degradation: mechanisms, strategies and application. Signal Transduct Target Ther. 2022;7(1):113. Epub 20220404. doi: 10.1038/s41392-022-00966-4. PubMed PMID: 35379777; PubMed Central PMCID: PMCPMC8977435.
Banaszynski LA, Chen LC, Maynard-Smith LA, Ooi AG, Wandless TJ. A rapid, reversible, and tunable method to regulate protein function in living cells using synthetic small molecules. Cell. 2006;126(5):995-1004. doi: 10.1016/j.cell.2006.07.025. PubMed PMID: 16959577; PubMed Central PMCID: PMCPMC3290523.
Dice JF. Peptide sequences that target cytosolic proteins for lysosomal proteolysis. Trends Biochem Sci. 1990;15(8):305-9. doi: 10.1016/0968-0004(90)90019-8. PubMed PMID: 2204156. Wing SS, Chiang HL, Goldberg AL, Dice JF. Proteins containing peptide sequences related to Lys-Phe-Glu-Arg-Gln are selectively depleted in liver and heart, but not skeletal muscle, of fasted rats. Biochem J. 1991;275 ( Pt 1):165-9. doi: 10.1042/bj2750165. PubMed PMID: 2018472; PubMed Central PMCID: PMCPMC1150027.
Kovac A, Miskey C, Menzel M, Grueso E, Gogol-Doring A, Ivics Z. RNA-guided retargeting of Sleeping Beauty transposition in human cells. Elife. 2020;9. Epub 20200306. doi: 10.7554/eLife.53868. PubMed PMID: 32142408; PubMed Central PMCID: PMCPMC7077980. Wilber et al., 2006, RNA as a source of transposase for Sleeping Beauty-mediated gene insertion and expression in somatic cells and tissues. Mol Ther. 13(3), 625-30.
Wilber et al., 2007, Messenger RNA as a source of transposase for sleeping beauty transposon-mediated correction of hereditary tyrosinemia type I. Mol Ther 15:1280-7. Cocchiarella F, Latella MC, Basile V, Miselli F, Galla M, Imbriano C, Recchia A. Transcriptionally regulated and nontoxic delivery of the hyperactive Sleeping Beauty Transposase. Mol Ther Methods Clin Dev. 2016 Jun 15;3:16038. doi: 10.1038/mtm.2016.38. PMID: 27574698; PMCID: PMC4985251.
Russell JE, Liebhaber SA. The stability of human beta-globin mRNA is dependent on structural determinants positioned within its 3' untranslated region. Blood. 1996 Jun 15;87(12):5314-23. PMID: 8652847.
Dikic I, Johansen T, Kirkin V. Selective autophagy in cancer development and therapy. Cancer Res. 2010 May 1;70(9):3431-4. doi: 10.1158/0008-5472.CAN-09-4027. Epub 2010 Apr 27. PMID: 20424122.
Mátés L, Chuah MK, Belay E, et al. Molecular evolution of a novel hyperactive Sleeping Beauty transposase enables robust stable gene transfer in vertebrates. Nat Genet. 2009 Jun;41(6):753-61. doi: 10.1038/ng.343. Epub 2009 May 3. PMID: 19412179.
Yadati T, Houben T, Bitorina A, Shiri-Sverdlov R. The Ins and Outs of Cathepsins: Physiological Function and Role in Disease Management. Cells. 2020 Jul 13;9(7):1679. doi: 10.3390/cells9071679. PMID: 32668602; PMCID: PMC7407943.
Gough NR, Hatem CL, Fambrough DM. The family of LAMP-2 proteins arises by alternative splicing from a single gene: characterization of the avian LAMP-2 gene and identification of mammalian homologs of LAMP-2b and LAMP-2c. DNA Cell Biol. 1995 Oct;14(10):863-7. doi: 10.1089/dna.1995.14.863. PMID: 7546292.
Fan X, Jin WY, Lu J, Wang J, Wang YT. Rapid and reversible knockdown of endogenous proteins by peptide-directed lysosomal degradation. Nat Neurosci. 2014 Mar;17(3):471-80. doi: 10.1038/nn.3637. Epub 2014 Jan 26. PMID: 24464042; PMCID: PMC3937121.

## Claims

1. A polypeptide comprising a *Tc1*/*mariner* superfamily transposase capable of mobilizing a transposon, wherein the polypeptide comprises a nuclear export signal and a chaperone-mediated autophagy signal and wherein the transposase is selected from the group comprising Sleeping Beauty, Frog Prince, Minos, ZB and Passport transposase, preferably, Sleeping Beauty transposase.

2. The polypeptide of claim 1, wherein the polypeptide comprises an SB100X transposase having an amino acid sequence having at least 80% sequence identity to SEQ ID NO: 43, wherein, preferably, the polypeptide further has an amino acid sequence having at least 80% sequence identity to SEQ ID NO: 50.

3. The polypeptide of any of the preceding claims, wherein the chaperone-mediated autophagy signal has an amino acid sequence selected from the group comprising SEQ ID NO: 31, 32, 33, 34, 35, 36, 37, 38, 39, 40, 41 and 42.

4. An RNA enconding the polypeptide of any of the preceding claims, wherein the RNA comprises a 5'UTR and a 3'UTR, wherein the 5'UTR is seleceted from the group comprising a β-globin 5'UTR and a 5'UTR comprising an elF4G aptamer.

5. The RNA of claim 4, wherein the 3'UTR is selected from the group consisting of one or more β-globin 3'UTRs, an α-globin 2 3'UTR, a 3'UTR comprising a WPRE, a βα-globin 3'UTR and a truncated β-globin 3'UTR.

6. The RNA of any of claims 4 or 5, wherein the RNA comprises
a) a β-globin 5'UTR and two β-globin 3'UTRs;
b) a β-globin 5'UTR and a truncated β-globin 3'UTR;
c) a β-globin 5'UTR and an α -globin 2 3'UTR
d) a β-globin 5'UTR and a βa-globin 3'UTR; or
e) a 5'UTR comprising an eIF4G aptamer and two β-globin 3'UTRs.

7. The RNA of any of claims 4 to 6, wherein the RNA is codon-optimized.

8. The RNA of any of claims 4 to 7, wherein the RNA comprises a poly-A tail having a length of 50 to 150 nt, preferably 75 to 100 nt, such as 90 nt.

9. The RNA of any of claims 4 to 8, wherein the RNA comprises one or more modified nucleosides selected from the group comprising 5-methyl-CTP, N4-acetyl-CTP, Pseudo-UTP, N1-methylpseudo-UTP, 6-methoxy-UTP, 2-thio-UTP, N6-methyl-ATP and N1-methyl-ATP.

10. The RNA of any of claims 4 to 9, wherein the RNA comprises a nucleic acid sequence of SEQ ID NO: 62, 63, 64, 65, 66, 67, 68, or 69, preferably SEQ ID NO: 69.

11. A vector for introducing the RNA of any of claims 4 to 10 and, optionally, the polypeptide of claims 1 to 3 into a target cell, wherein the vector comprises a nucleic acid encoding the RNA of any of claims 4 to 10 or the polypeptide of any of claims 1 to 3, or wherein the vector comprises the RNA of any of claims 4 to 10 or the polypeptide of any of claims 1 to 3.

12. A cell comprising the RNA of any of claims 4 to 10 and/or the polypeptide of any of claims 1-3; wherein, preferably, the cell is a human cell selected from the group comprising a pluripotent stem cell and a human lymphocyte, optionally, a human T lymphocyte.

13. A kit comprising
a) the polypeptide of any of claims 1 to 3, the RNA of any of claims 4 to 10, the vector of claim 11 and/or the cell of claim 12; and
b) a nucleic acid comprising a cargo nucleic acid flanked by terminal inverted repeats (TIR) of a transposon capable of being mobilized by said transposase.

14. A pharmaceutical composition comprising the polypeptide of any of claims 1-3, the RNA of any of claims 4 to 10; the vector of claim 11, the cell of claim 12, and/or the kit of claim 13 and, optionally, a pharmaceutically acceptable carrier and/or excipient, wherein the pharmaceutical composition preferably is for use in adoptive T cell therapy or gene therapy.

15. Use of the polypeptide of any of claims 1-3, the RNA of any of claims 4 to 10, the vector of claim 11, the cell of claim 12 or the kit of claim 13 for introducing an exogenous nucleic acid into the genome of a cell, wherein the use optionally is an in vitro use.

16. A method, optionally, an in vitro method, for preparing a cell with an exogenous nucleic acid integrated into the genome of the cell, comprising steps of
a) providing to the cell the polypeptide of any of claims 1-3 or the RNA of any of claims 4 to 10; and
b) providing to the cell a nucleic acid comprising the exogenous nucleic acid flanked by terminal inverted repeats (TIR) of a transposon capable of being mobilized by said polypeptide or a polypeptide encoded by said RNA.
